# EUROPEAN PATENT APPLICATION

(11) **EP 0 523 296 A1**
(43) Date of publication of application: **20.01.1993**
(21) Application number: 91306513.2
(22) Date of filing: 17.07.1991
(51) Int. Cl.: C07K 3/08

(54) **An improved method for folding tissue plasminogen activators and derivatives thereof**

(71) Applicant: ELI LILLY AND COMPANY, Indianapolis, Indiana 46285 (US)
(72) Inventor: Mackellar, Warren Cameron, Indianapolis, Indiana 46217 (US); Robey, Carolyn Sue, Indianapolis, Indiana 46219 (US)
(74) Representative: Hudson, Christopher Mark

(57) **Abstract**

The present invention discloses and claims the addition of lysine to protein folding procedures to improve the yield of substrate specific, enzymatically active tissue plasminogen activator or tissue plasminogen activator derivatives comprising kringle-2.

## Description

The relationship between the proper conformation of a protein and biological activity has long been appreciated. Anfinsen, 1973, Science 181:223-240, pioneered work in the area of protein folding with his work on ribonuclease. Anfinsen's research revealed the importance of proper disulfide bond formation on enzymatic activity in ribonuclease, the tertiary structure of which is dependent upon proper disulfide bond formation.

The genetic engineering of various genes for expression of their protein products in procaryotic cells has refocused much attention on the stringent conformational requirements of many proteins for maintenance of bioactivity. Many genes cloned into procaryotes have been efficiently expressed only to have the protein product form amorphous insoluble complexes with minimal or no bioactivity. Most often the insolubility and inactivity are resultant of improper disulfide linkages, which can be either inter-molecular or intra-molecular. Larger, more complex proteins having multiple domains depending on proper disulfide formation are especially prone to folding problems due to the possible permutations and combinations of the numerous cysteines involved.

A variety of approaches have been attempted to fold proteins into conformations appropriate for biological activity. Anfinsen's pioneering work determined that the denaturation and reduction of an enzyme yielded an inactive preparation even after the removal of denaturing and reducing agents. Anfinsen's chance finding that enzyme activity returned after prolonged periods was correctly perceived as being attributable to an oxidation of the reduced sulfhydryl groups and the accompanying folding to active form. Recent research in protein chemistry has greatly extended the art of protein folding. Procedures for protein folding include the use of a denaturing solution to linearize the polypeptide of interest followed by removal of the denaturant to allow folding. Solutions used for protein denaturation as well as those in which the proteins are allowed to refold are well documented in the scientific literature.

Myer, 1968, Biochem. 7:765 and Stellwagen, 1968, Biochem. 7:2893 reported the use of urea to denature proteins. Various approaches including: solid phase adsorption as described by Patchornik and Kraus, 1975, Pure Appl. Chem. 43:503; covalent attachment to insoluble carriers as disclosed by Sinha and Light, 1975, J. Biol. Chem. 250:8624 and Junolino, et al., 1978, Arch. Biochem. Biophys. 191:269; and adsorption to ion-exchange resins as described by Creighton, 1986, in Protein Structure, Folding, and Design, Oxender ed., Alan Liss, New York, p. 249-257 are alternatives to folding of proteins in solution and offer the theoretical advantage of minimizing inter-molecular bonding of cysteines. Solutions for protein folding are often a descending concentration gradient of the denaturing solution. The descending concentration gradient is generated either by dialysis or chromatographic procedures. The glutathione redox buffer solution reported by Ahmed, et al., 1975, J. Biol. Chem. 250:8477, has been used extensively with apparent success for many proteins.

The folding procedures in the prior art apparently work for many proteins. However, proteins such as tissue plasminogen activator with complex tertiary structures involving multiple disulfide bonds are not efficiently folded by the existing methods.

Tissue plasminogen activator (t-PA) converts plasminogen into plasmin. Plasmin enzymatically digests fibrin clots. t-PA as well as derivatives of t-PA are of medical and commercial importance in the treatment of homeostatic imbalances. When homeostasis is disrupted, fibrin clots may form resulting in myocardial infarction, deep vein thrombosis, stroke, and pulmonary embolism. Medical intervention with t-PA or t-PA derivatives offer immense promise in the treatment of the aforementioned homeostatic imbalances.

The complexity of t-PA's structure and the multiple disulfide bonding required for biological activity pose significant barriers to the proper folding of t-PA or derivatives of t-PA.

The present invention discloses and claims the surprising discovery that the addition of an effective amount of lysine promotes the proper folding of t-PA and its derivatives which contain kringle-2 and thus increases the efficiency of existing folding procedures.

A brief description of the drawings is provided below. The figures are not drawn precisely to scale. For convenience, some restrictions endonuclease sites which are not utilized in the constructions described in the Examples are not listed.

Figure 1 depicts the amino acid sequence of human t-PA.

Figure 2 is a restriction site and function map of plasmid ptPA102.

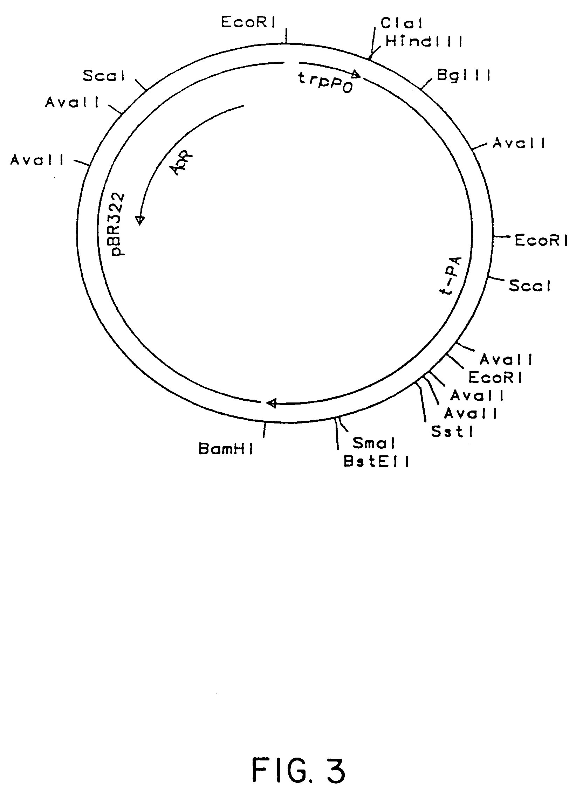

Figure 3 is a restriction site and function map of plasmid ptPA103.

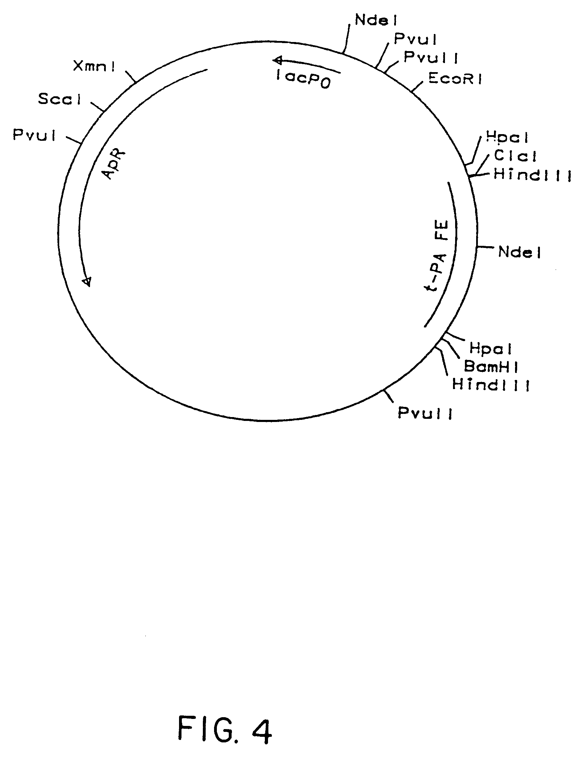

Figure 4 is a restriction site and function map of plasmid pUC19 TPAFE.

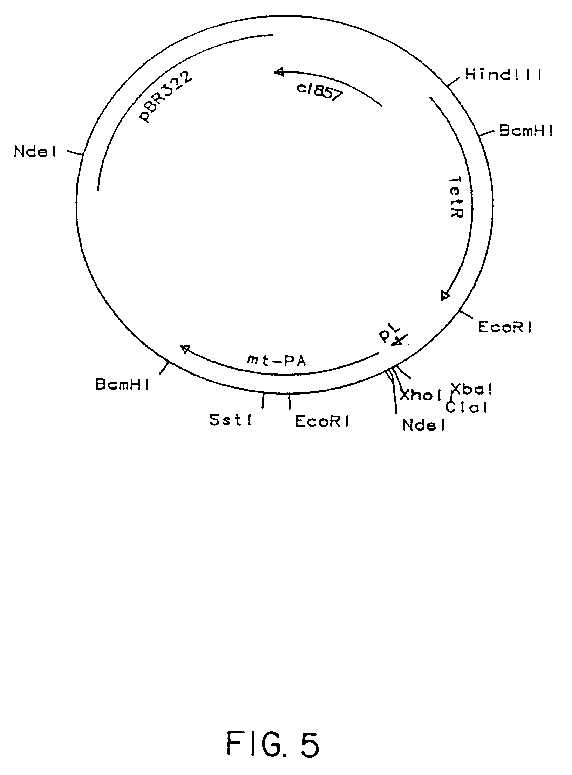

Figure 5 is a restriction site and function map of plasmid pBW28.

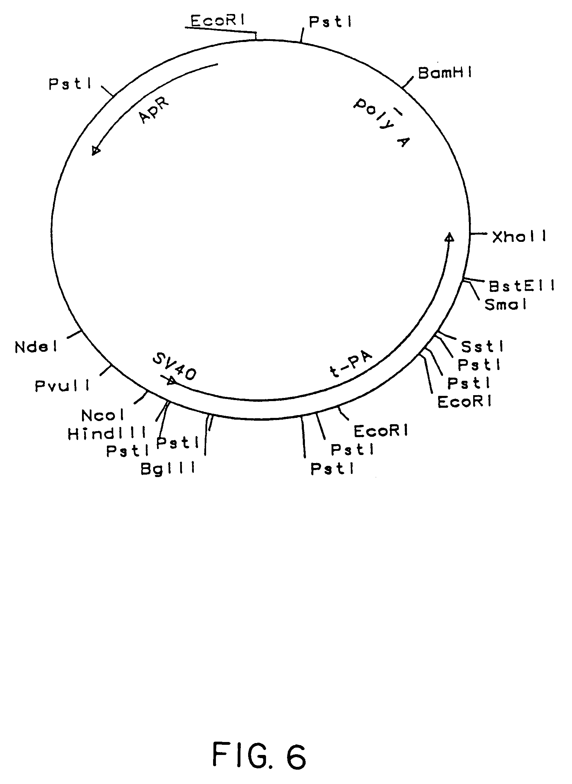

Figure 6 is a restriction site and function map of plasmid ptPA301.

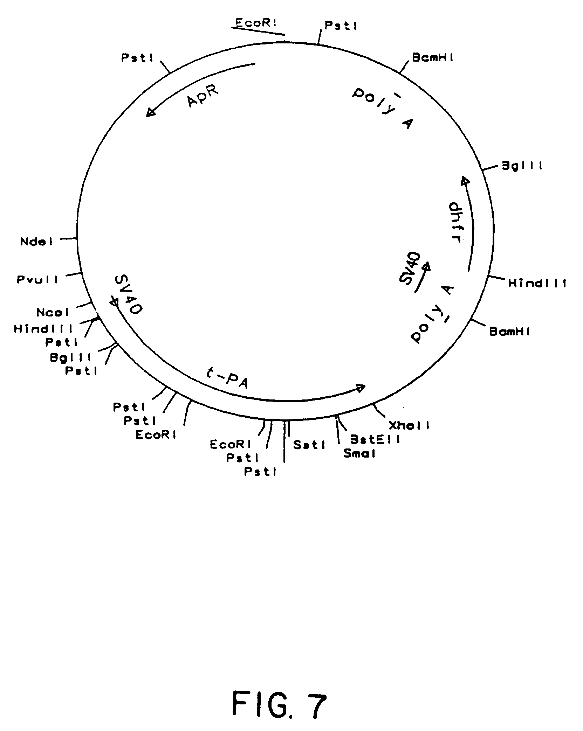

Figure 7 is a restriction site and function map of plasmid ptPA303.

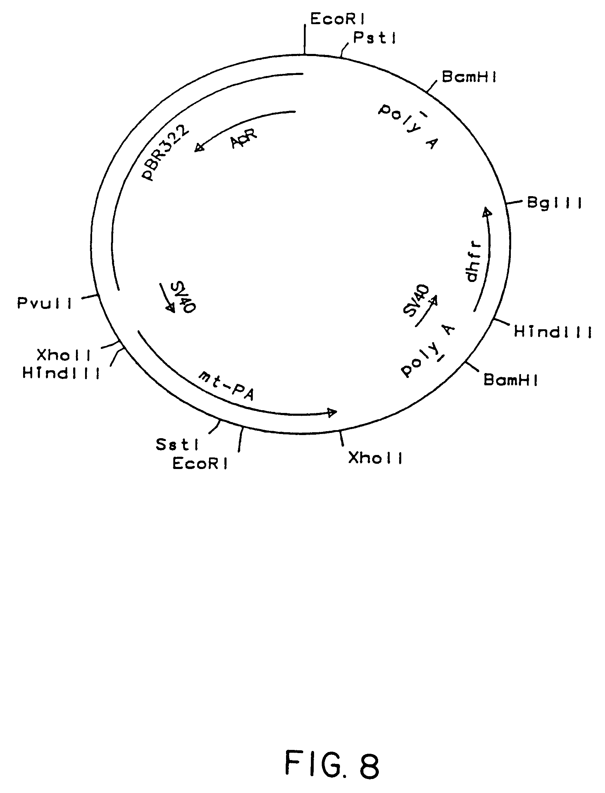

Figure 8 is a restriction site and function map of plasmid pBW32.

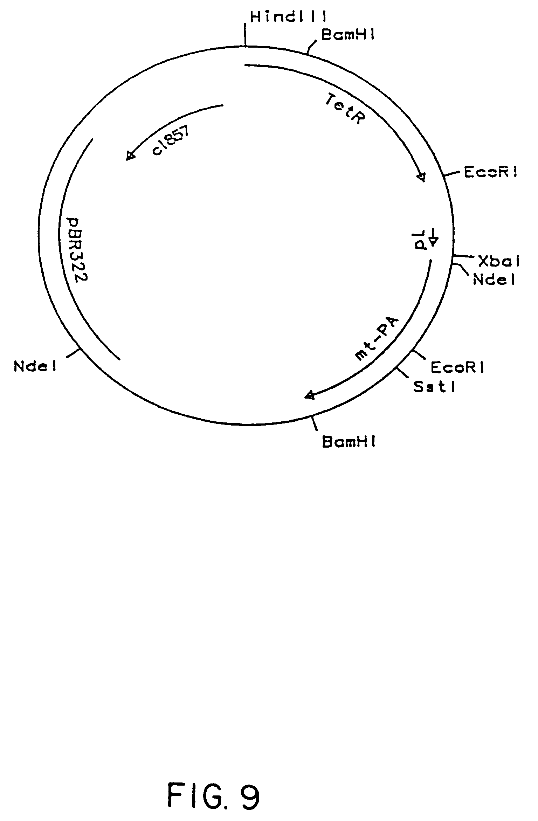

Figure 9 is a restriction site and function map of plasmid pBW33, 35, 36.

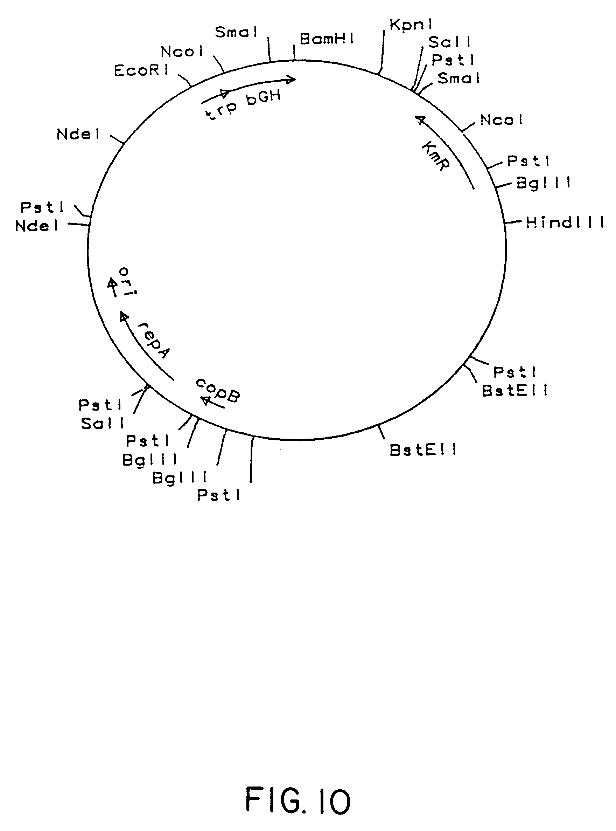

Figure 10 is a restriction site and function map of plasmid pCZ106.

Figure 11 is a restriction site and function map of plasmid pKC283.

Figure 12 is a restriction site and function map of plasmid pKS283-PX.

Figure 13 is a restriction site and function map of plasmid pKC283-L.

Figure 14 is a restriction site and function map of plasmid pKC283-LB.

Figure 15 is a restriction site and function map of plasmid pKC283-PRS.

Figure 16 is a restriction site and function map of plasmid pL32.

Figure 17 is a restriction site and function map of plasmid pL47.

Figure 18 is a restriction site and function map of plasmid pL84.

Figure 19 is a restriction site and function map of plasmid pL95.

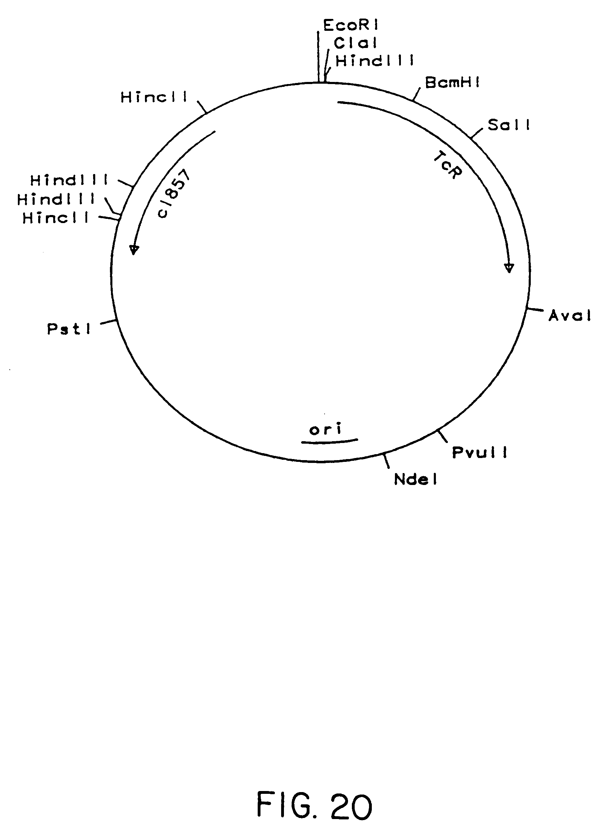

Figure 20 is a restriction site and function map of plasmid pR12.

Figure 21 is a restriction site and function map of plasmid pR12AR1.

Figure 22 is a restriction site and function map of plasmid pL110.

Figure 23 is a restriction site and function map of plasmid pCZ101.

Figure 24 is a restriction site and function map of plasmid pL219.

Figure 25 illustrates the effect of urea concentration in the second step of the two-step folding procedure on t-PA-6 activity.

The present invention provides an improved method for folding t-PA and t-PA derivatives comprising kringle-2 which comprises adding an effective amount of lysine to increase the yield of substrate specific, enzymatically active molecules. Presumably, the lysine binds to the lysine binding site of kringle-2 and thereby promotes the proper folding of the t-PA or t-PA derivative comprising kringle-2.

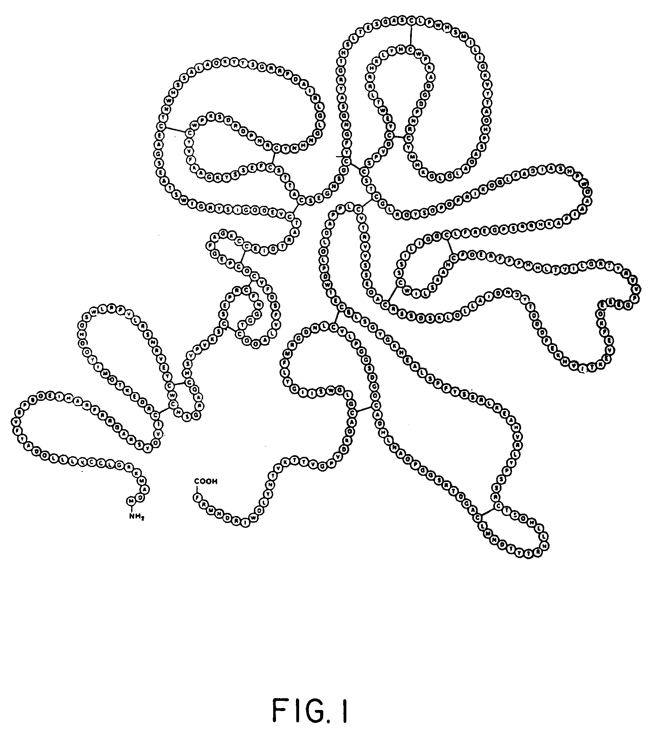

Figure 1 is a drawing of the amino acid sequence of human t-PA. Numerous biological activities are present in the t-PA molecule. The regions (domains) of the t-PA molecule which are associated with the various biological properties of t-PA are indicated in Figure 1. Several t-PA derivatives were constructed through genetic engineering in an effort to improve upon the therapeutic utility of t-PA. For example, in t-PA-3 both kringles (1 and 2 of Figure 1) were deleted. t-PA-4 does not contain kringle 1. t-PA-6 is missing the finger, epidermal growth factor, and kringle 1 domains. t-PA-3, t-PA-4, and t-PA-6 were constructed by site-specific mutagenesis of the t-PA coding region using m13 vectors and oligonucleotide sequences chemically synthesized to affect deletions of the regions specified above. The construction of expression vectors for t-PA-3, t-PA-4, and t-PA-6 is detailed in Examples 1-30. A t-PA derivative similar to t-PA-6 is disclosed and claimed in E.P.O. Publication number 0 234 051, published February 9, 1987. The present invention is applicable to the folding of substrate specific, enzymatically active t-PA derivatives as well as native t-PA.

The present invention embraces only an improved folding process for t-PA or t-PA derivatives, which comprise kringle-2 and which are expressed in procaryotic cells. However, the nature of the expression vectors used as a source of the starting materials for the folding process disclosed and claimed herein is described in detail to guide skilled artisans in their practice of the present invention.

Plasmid pBW33 is the preferred procaryotic expression vector for t-PA-3. Plasmid pBW33 construction is described in Example 10. Plasmid pL219 is the preferred procaryotic expression vector for t-PA-4. Construction of plasmid pL219 is taught in Example 29. Construction of plasmid pL231, the preferred procaryotic expression vector for t-PA-6, is taught in Example 31. The aforementioned procaryotic expression vectors differ in the species of t-PA derivative expressed, but share many features. Plasmids pBW33, pL219, and pL231 share the following features: a tetracycline resistance gene is used as a selectable marker; an E. coli origin of replication derived from plasmid pBR322 is used for replication, a lambda pL promoter drives expression of the t-PA derivative, and thermal induction of product expression is achieved by use of cI857, a temperature sensitive repressor. Thus, expression of any of the t-PA derivatives utilized to illustrate the method of the present invention requires only familiarity with microbiological techniques.

The granules of protein, which serve as the starting material for the folding process improvement, are produced by culturing the expression systems at approximately 32°C until the desired density of expression systems is attained followed by shifting the temperature to about 42°C. Increasing the temperature to ∼42°C causes thermal inactivation of the cI857 repressor and allows expression of the recombinant protein to be driven from the pL promoter. Accumulation of the t-PA derivative within the E. coli host cell results in formation of protein granules or inclusion bodies. The inclusion bodies comprise the recombinant protein of interest as well as various E. coli components.

Prior to folding the t-PA or t-PA derivatives it is necessary to solubilize the granules. Granules of t-PA or t-PA derivatives are suspended in distilled water (1 ml/g). Approximately 1 hour of gentle stirring at 4°C was sufficient for the preparation of a homogeneous suspension of protein granules comprising tissue-type plasminogen activator and the derivatives thereof used to exemplify the method of the present invention. The protein concentration of the solubilized granules was determined using Protein Assay Reagent Product No. 23200 (Pierce Chemical Co., Rockford, IL) and the procedure provided by the vendor. Sulfitolyzed and ion-exchange purified materials comprise the preferred starting materials for the folding steps. Details of the sulfitolysis procedure are provided in Example 32. Example 33 describes the ion-exchange purification of sulfitolyzed t-PA-6.

Folding of proteins produced through recombinant DNA technology has traditionally relied on denaturization of the protein using either guanidine hydrochloride or urea followed by dialysis against a non-denaturing solvent. Thus, the two-step method of the present invention, which merely involves diluting the step 1 denaturant solution to provide an environment conducive to further folding is a significant advance in the art of protein folding.

A two-step folding procedure is illustrated in Example 34. The two-step method comprises preparing a solution of the protein in approximately 7M urea then diluting the step 1 (∼7M urea) solution to provide an environment conducive to further folding. Example 34 teaches the use of the two-step method to fold t-PA-6. The broad utility of the two-step method for other t-PA derivatives was confirmed by the folding of t-PA-3 and t-PA-4 at efficiencies similar to those obtained by dialysis. The two-step folding process of Example 34 results in 10-15% of the folded t-PA-6 being in enzymatically active form.

A comparison of fold efficiencies using the two-step method versus dialysis is provided in Example 35. Results of the comparison are set forth in Table 1 below.

**Table 1**

| Experiment | U/ML Two-Step Method of Ex. 34 | Dialysis |
|---|---|---|
| 1 | 402 | 411 |
| 2 | 443 | 468 |
| 3 | 411 | 420 |
| Mean | 419 | 433 |

The ability of the two-step method to achieve fold efficiencies comparable to those obtained by dialysis are apparent in Table 1. Simplification of the folding procedure to a two-step, dilution based approach offers significant advantages over dialysis. The use of dialysis type (diafiltration) buffer exchange equipment in industrial scale dialysis frequently generates hydrodynamic shear forces, thus compromising the recovery of biologically active material. Thus, the two-step method is a significant advance in the art of protein folding. Furthermore, the two-step method is broadly applicable for folding t-PA derivatives as illustrated by the successful folding of t-PA-4 and t-PA-6.

The urea concentrations in steps 1 and 2 were varied to determine the preferred urea concentrations in each of these steps. Example 36A provides the experimental methodology utilized to generate an optimal step 1 urea concentration. A 7M urea concentration in step 1 resulted in 490 u/ml of t-PA activity, while a 4M urea concentration resulted in 23 u/ml of t-PA activity and a 1M urea concentration in Step 1 yielded undetectable levels of t-PA activity after completion of the standard second step taught in Example 34. Thus, the preferred urea concentration in step 1 is from about 5M to about 8M urea with approximately 7M being especially preferred.

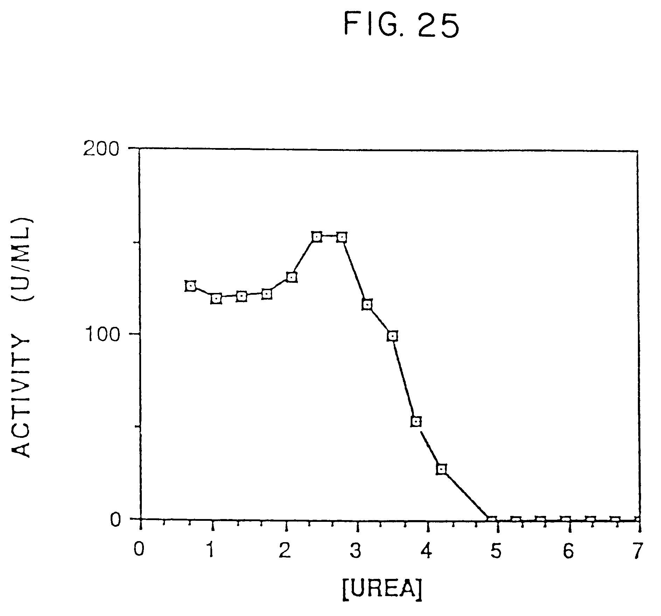

Figure 25 further illustrates the effect of urea concentrations on folding. Samples of t-PA derivatives were assayed directly from the folding mixture for fibrinolytic activity on fibrin plates. Samples were removed at each of the time points and potassium tetrathionate was added to the samples to prevent further disulfide rearrangement. The data of Figure 25 illustrates that a urea concentration in the range of 1.5M to 3.5M is the preferred urea concentration for the second step. Thus, a 1:2 or a 1:3 dilution of the step 1 solution is preferred to initiate step 2 of the folding procedure.

Folding under preferred urea concentration is completed by about 48 hours. Thus, a time period of about 48 hours is preferred for the second step of the folding.

The complex disulfide bonding required for substrate specific, enzymatically active t-PA or t-PA derivatives is achieved in the two-step method by providing two separate folding environments. The two folding environments, approximately 7M urea and approximately 1.5M-3.5M urea respectively, allow the sequential folding of domains of the t-PA or t-PA derivatives under conditions more conducive to the folding of that particular domain.

Thus, the method of the present invention--when practiced using the two-step method--comprises (a) preparing a solution of t-PA or t-PA derivative in a sufficient concentration of urea to disrupt secondary and tertiary structure of said t-PA or t-PA derivative, and (b) diluting the step (a) solution to yield a second environment conducive to the further folding of the t-PA or t-PA derivative into substrate specific, enzymatically active molecules. As used in the present invention, the phrase "disrupt secondary and tertiary structure" does not mean that secondary and tertiary structure is totally eliminated. Disruption of secondary and tertiary structure is necessary to achieve the transition state required to initiate proper domain folding.

The addition of an agent to terminate disulfide bond rearrangement is of importance in defining optimal refolding conditions. When samples were removed from the folding process, diluted, and assayed for fibrinolytic activity, an artifactual elevated activity was noticed in samples removed from high urea concentrations. It was determined that the dilution necessary for fibrinolytic assay reduced the urea concentration sufficiently for some folding to occur during the course of the fibrinolytic assay. The addition of potassium tetrathionate allows an accurate determination of the activity of the samples. Fibrinolytic activity was measured in substantial accordance with the fibrin plate method of Astrop and Mullertz, 1952, Arch. Biochem. 40:346. The fold efficiencies are based on the fibrinolytic activity as determined in the fibrin plate assay using a 200,000 u/mg of protein conversion. Protein concentrations were determined by absorbance measurements derived from scanning Coomassie blue stained SDS polyacrylamide gels with a laser densitometer.

A further aspect of the present invention is the addition of Tween to the second step of the folding process. The preferred concentration of Tween was determined using a two-step fold procedure in which various concentrations of Tween 20 (polyoxyethylene-sorbitan monolaurate) and Tween 80 (polyoxyethylene-sorbitan monooleate) were evaluated. Samples of t-PA-6 were folded using a two-step method with various concentrations of Tween included in the second step. Ion-exchange purified and sulfitolyzed t-PA-6 was diluted in step 1 buffer: 7M urea, 50mM Tris, 0.15M lysine monohydrochloride, 7mM cysteine monohydrochloride, 0.2M NaCl, 1mM Na₂ EDTA, pH 9.2 and incubated for about 16 hours. The second step was performed by diluting the step 1 mixture 1:3 with step 2 buffer: 50mM Tris, 80mM lysine monohydrochloride, 2mM cysteine monohydrochloride, pH 9.2. When the dilution commencing the second step was performed, various amounts of either Tween 20 or Tween 80 were added to the second step folding solution to generate a range of Tween concentrations. The folding was allowed to proceed for 48 hours, at which point samples were removed, treated with potassium tetrathionate to terminate the folding, and assayed for fibrinolytic activity.

Both Tween 20 and Tween 80 promoted the proper folding of proteins. Concentrations of Tween in the range of 0.3% to 1.0 are preferred in the second step of the present invention.

The addition of lysine to the folding solutions of the two-step folding process for t-PA or t-PA derivatives which comprise kringle-2, resulted in a significant and unexpected increase in the yield of substrate specific, enzymatically active material. The effect of lysine on t-PA folding is not a general phenomenon associated with cationic amino acids as arginine did not significantly increase the yield of fibrinolytically active t-PA or t-PA derivatives. Results of an experiment illustrating this effect are set forth in Table II below.

**Table 2**

| Effect of Lysine and Arginine on t-PA Derivative Folding | |
|---|---|
| Amino Acid addition (Step 1) | Final Activity (u/ml in final step 2) |
| None | 177 |
| Lysine (0.15M) | 490 |
| Arginine (0.15M) | 183 |

The data of Table II was obtained by folding t-PA-6 in substantial accordance with the 2-step folding procedure taught in Example 34 except that lysine was included at 0.15M in step 1. A 1:3 dilution of step 1 was done to initiate step 2. The presence of lysine is a preferred embodiment of the two-step folding procedure. The preferred concentration of lysine in step 2 of the folding procedure is from about 0.01M to about 1.5M with from about 0.020 to about 1.0M being more preferred and a lysine concentration of about 0.13M being especially preferred.

The preferred concentrations of lysine as set forth above are the preferred concentrations regardless of which t-PA derivative is being folded. Skilled artisans will realize that lysine will promote folding of t-PA and t-PA derivatives regardless of whether the two-step method, diafiltration or dialysis is used to lower the denaturant concentration.
The only criticality to the use of lysine to promote folding of t-PA or a t-PA derivative which comprises kringle-2 is that the lysine be present at an effective concentration during the folding of the molecule into a substrate specific, enzymatically active form.

### Example 1

Culture of E. coli MM294/pTPA102
and Isolation of Plasmid pTPA102
A.   Culture of E. coli MM294/pTPA102

One liter of M9 medium (Maniatis et al., 1982, Molecular Cloning, Cold Spring Harbor Laboratory) containing 0.2% casamino acids and 100 µg/ml ampicillin was inoculated with a culture of E. coli MM294/pTPA102 (NRRL B-15834) and incubated with shaking at 37°C until the optical density (O.D.) at 590 nm was ∼0.5 absorbance unit. At that time, chloramphenicol powder was added to a final concentration of 250 mg/ml and incubation continued overnight.

### B. Isolation of Plasmid pTPA102

E. coli MM294/pTPA102 were harvested from the culture described in Example 1A following a procedure adapted from Maniatis et al., 1982.

The cells were harvested by centrifugation at 4000 g for 10 minutes at 4°C, and the supernatant was discarded. The cell pellet was washed in 100 ml of ice-cold STE buffer (0.1 M NaCl; 10 mM Tris-HCl, pH 7.8; and 1 mM EDTA). Tris is an abbreviation of Tris (hydroxymethyl)aminomethane. EDTA is an abbreviation of ethylenediaminetetraacetic acid. After washing, the cell pellet was resuspended in 10 ml of Solution 1 (50 mM glucose; 25 mM Tris-HCl, pH 8.0; and 10 mM EDTA) containing 5 mg/ml lysozyme and left at room temperature for 10 minutes. Twenty ml of Solution 2 (0.2 N NaOH and 1% SDS) were then added to the lysozyme-treated cells, and the solution was gently mixed by inversion. The mixture was incubated on ice for 10 minutes.

Fifteen ml of ice-cold 5 M potassium acetate, pH 4.8, were added to the lysed-cell mixture and the solution mixed by inversion. The solution was incubated on ice for 10 minutes. The 5 M potassium acetate solution was prepared by adding 11.5 ml of glacial acetic acid to 28.5 ml of water and 60 ml of 5 M potassium acetate; the resulting solution was 3 M with respect to potassium and 5 M with respect to acetate.

The lysed cell mixture was centrifuged in a Beckman SW27 at 20,000 rpm for 20 minutes at 4°C. The cell DNA and debris formed a pellet on the bottom of the tube. About 36 ml of supernatant were recovered, and 0.6 volumes of isopropanol were added, mixed, and the resulting solution left at room temperature for 15 minutes. The plasmid DNA was collected by centrifugation at 12,000 g for 30 minutes at room temperature. The supernatant was discarded, and the DNA pellet was washed with 70% ethanol at room temperature. The ethanol wash was decanted, and the pellet was dried in a vacuum desiccator. The pellet was then resuspended in 8 ml of TE buffer. (10 mM Tris-HCl, 1 mM EDTA, pH 7.4).

Eight grams of CsCl were added to the DNA solution. About 0.8 ml of a 10 mg/ml solution of ethidium bromide in water were added for each 10 ml of CsCl-DNA solution. The final density of the solution was about 1.55 g/ml, and the ethidium bromide concentration was about 600 µg/ml. The solution was transferred to a Beckman Type 50 centrifuge tube, which was then filled to the top with paraffin oil, sealed, and centrifuged at 45,000 rpm for 24 hours at 20°C. After centrifugation, two bands of DNA were visible in ordinary light. After removing the cap from the tube, the lower DNA band was removed by using a syringe with a #21 hypodermic needle inserted through the side of the centrifuge tube.

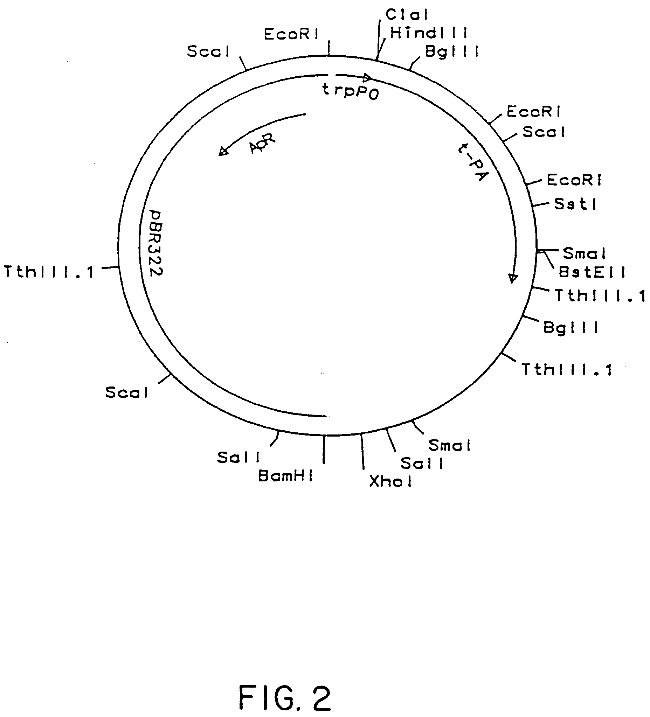

The ethidium bromide was removed by several extractions with water-saturated 1-butanol. The CsCl was removed by dialysis against TE buffer. After extractions with buffered phenol and then chloroform, the DNA was precipitated, washed with 70% ethanol, and dried. About 1 mg of plasmid pTPA102 was obtained and stored at 4°C in TE buffer at a concentration of about 1 µg/ul. A restriction site and function map of plasmid pTPA102 is presented in Figure 2 of the accompanying drawings.

### Example 2

### A. Construction of Intermediate Plasmid pTPA103

About 50 µg of plasmid pTPA102 isolated from E. coli K12 MM294 cells (NRRL B-15625) were digested to completion with 50 units of TthIII.1 restriction enzyme* in restriction buffer A (100 mM NaCl, 10 mM Tris-HCl pH 8, 10 mM MgCl₂, 1 mM dithiothreitol) at 37°C. The digestion yielded 3 fragments; 4.4 kb, 3.1 kb, and 0.5 kb in size. The separated fragments were located in an agarose gel by staining with ethidium bromide and visualizing fluorescent bands with ultraviolet light. A slice containing the 4.4 kb fragment was cut out with a razor blade and DEAE membrane (NA45, Schleicher and Schuell, Inc.) was inserted into the slit. The gel slice was then returned to its original position. Electrophoresis was continued until the DNA migrated onto the DEAE membrane. The position of the membrane containing the DNA was cut out and washed 3X with 200 µl of 150 mM NaCl in 20 mM Tris-HCl pH 8 in a 0.5 ml Eppendorf tube. The DNA was eluted off the membrane with 200 µl 1M NaCl in 20 mM Tris-HCl pH 8 at 65°C. The DNA was then precipitated with 2.5 volumes 95% ethanol, resuspended in 0.3M NaOAc, reprecipitated and collected by centrifugation.
* Restriction enzymes and instructions can be obtained from the following sources:
1. New England Biolabs., Inc., 32 Tozer Road, Beverly, Massachusetts 01915
2. Boehringer-Mannheim Biochemicals, 7941 Castleway Drive, Indianapolis, Indiana 46250
3. Bethesda Research Laboratories Inc., 8717 Grovemont Circle, Gaithersburg, Maryland 20760
Hereinafter, New England Biolabs, Boehringer-Mannheim, and Bethesda Research Labs will be referred to as NEB, BMB, and BRL, respectively.

About 5 µg of the DNA pellet was resuspended in 100 µl of nick-translation buffer (NTB; 50 mM Tris-HCl pH 7.2, 10 mM MgSO₄, 0.1 mM DTT) to which 1 µl of each dNTP (10 mM dATP, dTTP, dCTP, dGTP) and 10 units of Klenow enzyme, which is the large fragment of E. coli DNA polymerase I, (Boehringer Mannheim) were added. DTT is an abbreviation of dithiothreitol. dNTP is an abbreviation of deoxynucleotide triphosphates. The reaction was carried out at room temperature (20°C) for 30 minutes and terminated by heat treatment at 65°C for 5 minutes. BamHI linkers (5'-CGGATCCG-3' from New England Biolabs) were kinased and ligated to the 4.4 kb fragment.

The resulting ligation mix (20 µl) was diluted into 100 µl 1X A restriction buffer and 10 units of BamHI and 1 unit of HindIII restriction enzyme were added. Incubation was at 37°C until a ∼2.4 kb and a ∼2.0 kb fragment were generated. The fragments were separated on a 1% agarose gel and the ∼2.0 kb fragment was gel-purified as taught above, and then ligated between the HindIII and BamHI sites of plasmid pRC.

Before transformation, the ligation mix (20 µl) was diluted into 100 µl of restriction buffer A and 2 µl of NcoI restriction enzyme was added prior to incubation at 37°C for 2 hours. This treatment eliminates undesired recombinants.

### B. Transformation

The resultant ligation mixture was used to transform, in substantial accordance with the transformation procedure of Wensink, 1974, Cell 3:315, E. coli K12 RV308 on TY plates (1% tryptone, 0.5% yeast extract, 0.5% sodium chloride, 1.5% agar, pH 7.4) containing 50 µg/ml of ampicillin. Bacterial strain E. coli K12 RV308 has been deposited and made part of the permanent stock culture collection of the Northern Regional Research Laboratory, Peoria, Illinois, from which it is available to the public under the accession number NRRL B-15624.

The transformants were identified by their ampicillin resistant and kanamycin sensitive phenotype and by restriction enzyme analysis of plasmid DNA. The resultant cells were used to isolate plasmid pTPA103 in substantial accordance with the procedure of Example 1.

C.    Construction of plasmid pTPA103 der NdeI

About 1 µg of plasmid pTPA103 in 20 µl of A restriction buffer was digested at 37°C with restriction enzyme BglII to produce linears. The linearized plasmid was precipitated with 2.5 volumes of ethanol in the presence of 0.3M NaOAc and the DNA was collected by centrifugation. The DNA pellet was resuspended in 100 µl nick-translation buffer to which 1 µl of a 10mM solution of dNTP each (10 mM dATP, dTTP, dCTP, and dGTP) and 10 units of Klenow enzyme (BMB) were added. The reaction was carried out at room temperature (20°C) for 30 minutes and terminated by heat treatment at 65° for 5 min.). About 0.1 µg of NdeI linkers (5'-CCATATGG-3', NEB) were heated to 90°C in 10 µl of TE buffer (10 mM Tris-HCl pH 8; 1 mM EDTA) and reannealed by slowly lowering the temperature to 15°C. The linkers were added to the "filled-in" BglII fragment, in the presence of 1 µl T4 DNA ligase (40,000 units/ml) and ATP (0.5 mM final concentration). Ligation was at 15°C for 20 hours. The ligation mix was then transformed into competent E. coli K12 RV308 cells. Transformants were identified by their ampicillin resistant phenotype and by restriction enzyme analysis. DNA sequencing confirmed that pTPA103 der NdeI contained the following sequence:
5'..... CCATATGGGATCTTAC ... 3'

### Example 3

### Construction of Plasmid pUC19 TPA FE

About 10 µg of plasmid pTPA103 der NdeI was digested in 100 µl of restriction enzyme buffer A with 20 units AvaII at 37°C for 5 hours. The resulting restriction fragments were separated on a 1% agarose gel and the ∼1400 bp fragment was purified according to the teaching of Example 2A. About 1 µg of the purified 1400 bp fragment in 20 µl NTB buffer was treated with Klenow enzyme and 55 picomoles of kinased HpaI linkers (5'-CGTTAACG-3') were ligated in 100 µl of ligation buffer according to the teaching in Example 2.

The DNA fragment used to construct the linker can be synthesized either by using a Systec 1450A DNA Synthesizer (Systec Inc., 3816 Chandler Drive, Minneapolis, MN) or an ABS 380A DNA Synthesizer (Applied Biosystems, Inc., 850 Lincoln Centre Drive, Foster City, CA 94404). Many DNA synthesizing instruments are known in the art and can be used to make the fragment. In addition, the fragment can also be conventionally prepared in substantial accordance with the procedures of Itakura et al., 1977, Science, 198:1056 and Crea et al., 1978, Proc. Natl. Acad. Sci. USA, 75:5765. After ligation, 10 µl of 1M Tris-HCl pH 8 and 5 units of EcoRI restriction enzyme were added and the digestion was carried out at 37°C until complete. The sample was treated at 65°C for 5 minutes and the DNA was precipitated with 2.5 volumes of 95% ethanol in the presence of 0.3M NaOAc. The DNA was collected by centrifugation and resuspended in 10 µl of ligation buffer.

About 5 µg of plasmid pUC19 (Pharmacia, Inc., 800 Centennial Dr., Piscataway, NJ) were digested to completion at 37°C with SmaI restriction enzyme in 100 µl restriction buffer containing 10 mM Tris-HCl, pH 8, 10 mM MgCl₂, 1 mM β-mercapthoethanol and 20 mM KCl. About 10 µl of 1M Tris-HCl pH 8 and 10 units of EcoRI restriction enzyme were added and incubation was continued at 37°C until the digestion was complete. The digested plasmid DNA was run on a 1% agarose gel and the large EcoRI to SmaI restriction fragment was gel-purified. About 1 µg of the gel-purified fragment was dissolved in 10 µl ligation buffer and mixed with the 770 bp fragment from pTPA103 der NdeI prepared as described above. Two µl of 0.5 mM ATP and 1 µl of T4 DNA ligase (40,000 units/ml; NEB) were added and ligation carried out at 15°C for 2 hours. The ligation mix was diluted into 100 µl of ligase buffer containing 500 mM ATP and 40 units of T4 DNA ligase and kept at 23°C for 2 hours.

About 10 µl of the ligation mix was transformed into competent E. coli K12 RR1ΔM15 cells (NRRL B-15440) which were plated on TY agar plates containing 100 µg/ml ampicillin, 40 µg/ml X-gal (Sigma) and 1mM IPTG (Sigma). The plates were incubated at 37°C overnight and the transformants were identified by their inability to utilize the X-gal resulting in a "white" colony phenotype. These colonies were grown in TY broth containing 100 µg/ml ampicillin and plasmid DNA was extracted for restriction analysis. The desired plasmid had an insert of about 770 bp and new restriction sites for NdeI and HpaI. The plasmid, designated pUC19 TPA FE, contains the first 96 amino acid residues of native t-PA but, due to the addition of the translational activating sequence, the amino acid sequence has been renumbered to include the amino acid residues MET and GLY as amino acid residue numbers 1 and 2, respectively.

### Example 4

### Construction of Plasmid pBW25

A.    Isolation of pUC19 TPA FE HpaI-BamHI Fragment

Approximately 7 µg of pUC19 TPA FE were digested with ∼18 units of HpaI restriction enzyme in 100 µl of HpaI buffer for twenty minutes at 37°C in order to achieve only partial digestion as determined by analytical gel electrophoresis. The reaction salt concentration was adjusted to 150 µl with BamHI buffer (150 mM NaCl, 10 mM Tris pH 8.0, 10 mM MgCl₂) and ∼16 units of BamHI restriction enzyme were added. This reaction was incubated at 37°C for 90 minutes. DNA was concentrated by ethanol precipitation and electrophoresed on a preparative 1.5% agarose gel. The gel was stained in 1 µg/ml ethidium bromide and DNA bands were visualized under long wave UV light. The appropriate large HpaI-BamHI band (as determined by comparison to a complete HpaI-BamHI digest and intact plasmid) was excised and frozen for one hour at -20°C. DNA was recovered by the method of freeze-squeeze (Thuring et al., 1975, Anal. Biochem. 66:213) and precipitated twice out of 0.3M NaOAc with 2.5 volumes of 95% ethanol. The final pellet was rinsed twice with 70% ethanol, dried under vacuum and resuspended in 20 µl distilled water.

B.    Isolation of the ~1015 bp ScaI-BamHI Fragment of
Plasmid pTPA103

About 13 µg of pTPA103 were digested with 18 units of ScaI restriction enzyme in 100 µl ScaI buffer (100 mM NaCl, 10 mM Tris pH 7.5, 10 mM MgCl₂) at 37°C for 90 minutes. The reaction was adjusted to 150 µl with BamHI buffer and ∼16 units of BamHI restriction enzyme were added and the reaction incubated at 37°C for 90 minutes. DNA was concentrated by ethanol precipitation prior to loading on a 1.5% agarose preparative gel. The ∼1015 bp ScaI-BamHI band was visualized, excised and recovered as described in Example 4A.

### C. Ligation

About 100 ng of each of the above fragments described in Examples 4A and 4B were ligated in a 20 µl reaction mix containing 50 mM Tris-HCl pH 7.5, 10 mM MgCl₂, 10 mM DTT, 6 µg BSA and 1 mM ATP with 1 Weiss unit ligase (Promega Biotech) for 18 hours at 16°C. BSA is an abbreviation of bovine serum albumin.

D.   Transformation into E. coli MM294

The above ligation mix was diluted to 50 µl with 50 mM NaCl-10 mM MgCl₂-10 mM CaCl₂ and used to transform 100 µl freshly prepared competent E. coli MM294 cells in accordance with the teaching of Example 2B. The transformation mix was plated onto BHI (Brain Heart Infusion - Difco) plates containing 100 µg/ml ampicillin. Transformants were identified by their ampicillin resistant phenotype and restriction enzyme analysis of plasmid DNA, including the presence of an ∼1079 bp EcoRI fragment.

E.    Culture of E. coli MM294/pBW25 and Isolation of
Plasmid pBW25

E. coli MM294/pBW25 was cultured essentially as described in Example 1A except that BHI broth was used instead of TY.

Plasmid isolation was performed essentially as taught in Example 1B except that one discontinuous CsCl gradient (density = 1.80 g/ml and 1.47 g/ml) was performed instead of two successive homogeneous gradients (Garger et al., 1983, Biochemical and Biophysical Res. Comm., 117(3):835).

### Example 5

### Construction of Plasmid pM8BW26

A.    Isolation of the ~810 bp HindIII-EcoRI Fragment
of Plasmid pBW25

About 5 µg of plasmid pBW25 were digested with 10 units HindIII in 150 µl 1X HindIII buffer (50 mM NaCl, 10 mM Tris-HCl pH 8.0, 10 mM NaCl₂) at 37°C for 90 minutes. Fifteen µl 1M Tris-HCl pH 7.6 and 24 units EcoRI were added and incubation at 37°C continued for 90 minutes. The DNA was concentrated by ethanol precipitation and electrophoresed on a 1.5% agarose preparative gel. The ∼810 bp fragment was visualized and recovered as described in Example 4A.

B.    Isolation of the ~7.2 kb EcoRI-HindIII M13mp8 Vector

About 4.5 µg M13mp8 DNA (available from NEB) was digested as described in Example 5A. The ∼7.2 kb EcoRI-HindIII vector fragment was electrophoresed, identified and recovered as described in Example 4A.

C.    Ligation and Transfection of E. coli JM103

About 0.1 µg each of the EcoRI-HindIII fragment from pBW25 and the EcoRI-HindIII vector from M13mp8 were ligated as described in Example 4C except that the reaction temperature was 14°C. E. coli JM103 cells (BRL) were made competent and transfected with the ligation mix essentially as described in the BRL M13 Cloning/'Dideoxy' Sequencing Instruction Manual except that the amount of DNA used per transfection was varied. Recombinant plaques were identified by insertional inactivation of β-galactosidase activity (white plaque formation) and verified by restriction digests of RF DNA. For screening purposes, six white plaques were picked into 2.5 ml TY broth to which was added 0.4 mls log phase JM103 (TY broth culture was inoculated from minimal media stock to insure retention of F episome which also carries proAB). Cultures were incubated at 37°C with shaking for 8 hours. Cells from 1.5 ml aliquots were pelleted and RF DNA isolated in substantial accordance with the teaching of the alkaline miniscreen procedure of Birnboim and Doly, 1979, Nuc. Acid Res. 7:1513. The remainder of each culture was stored at 4°C for stock.

### D. Culture of JM103/pM8BW26 and Isolation of Single Stranded pM8BW26

Fifty ml log phase JM103 were infected with appropriate stock (described in Example 5C) and incubated at 37°C with shaking for 18 hours. Cells were pelleted by low speed centrifugation and single stranded DNA prepared from the culture supernatant by scaling up the procedure given in the aforementioned manual.

### Example 6

### Site Specific Mutagenesis to Construct Plasmid pM8BW27

### A. Mutagenesis

Single-stranded pM8BW26 was mutagenized in substantial accordance with the teaching of Adelman et al., 1983, DNA 2(3): 183-193, except that the Klenow reaction was done at room temperature for 30 minutes, followed by 60 minutes at 37°C, followed by 10°C for 18 hours. In addition, the S1 treatment was done at 20°C for 5 minutes, the salt concentration was reduced in half and the M13 sequencing primer (BRL) was used. The synthetic oligodeoxyribonucleotide primer used was 5'-GGGAAGTGCTGTGAAATATCCACCTGCGGCCTGAGA-3' to delete amino acid residues 87 through 261 of the native t-PA.

### B. Transfection of JM103 and Identification of Recombinants

The resulting mutagenesis mix was used to transfect JM103 as taught in Example 5C. Desired mutants were identified by restriction digest analysis of RF DNA and by Maxam and Gilbert DNA sequencing.

### Example 7

### Construction of Plasmid pBW28

A.   Isolation of the ~689 bp EcoRI-BamHI Fragment from
      Plasmid pTPA103.

Plasmid pTPA103 was digested to completion in 1X BamHI buffer with BamHI restriction enzyme. The buffer concentration was adjusted to accommodate EcoRI digestion as taught in Example 5A. The fragment was isolated and recovered in substantial accordance with the teaching in Example 4A.

B.    Isolation of XbaI-BamHI Vector of Plasmid pL110

Plasmid pL110 (the construction of pL110 is disclosed in subsequent examples) was partially digested with BamHI restriction enzyme. The reaction was extracted once with equal volume chloroform and the aqueous phase was then precipitated with ethanol. The recovered DNA was then digested to completion with XbaI restriction enzyme in 1X XbaI buffer (150 mM NaCl, 10 mM Tris HCl pH 7.9, 10 mM MgCl₂) at 37°C. The ∼5900 bp XbaI-BamHI linear fragment was isolated and recovered in accordance with the teaching in Example 4A.

### C. Construction of a Double Cistron

A linker can be synthesized as taught in Example 3A. The sequence of this fragment is as follows:

D.    Isolation of the ~563 bp NdeI-EcoRI Fragment from
Plasmid pM8BW27

XbaI
5'-CTAGAGGGTATTAATAATGTATCGATTTAAATAAGGAGGAATAACA-3'
TCCCATAATTATTACATAGCTAAATTTATTCCTCCTTATTGTAT
NdeI

RF pM8BW27 was digested with NdeI restriction enzyme, followed by EcoRI restriction enzyme, in substantial accordance with the teaching of Example 7A, except that the enzyme NdeI was substituted for BamHI. The fragment was isolated and recovered in substantial accordance with the teaching in Example 4A.

E.    Ligation and Construction of E. coli MM294/pBW28

About 50 ng of each of the above fragments described in Examples A through D were ligated and E. coli MM294 transformed in substantial accordance with the teaching of Example 4C and 4D. Transformants were identified by their tetracycline resistant phenotype and restriction enzyme analysis of plasmid DNA.

### Example 8

### Construction of Plasmid pTPA303

A.    Isolation of the ~4.2 kb BglII-HindIII Restriction
       Fragment of Plasmid pSV2-β-globin

Approximately 10 µg of plasmid pSV2-β-globin DNA (NRRL B-15928) were dissolved in 10 µl 10X HindIII reaction buffer, 5 µl (∼50 units) restriction enzyme HindIII, and 85 µl H₂0, and the reaction was placed at 37°C for 2 hours. The reaction mixture was then made 0.15 M in LiCl, and after adding 2.5 volumes of ethanol and chilling in a dry ice-ethanol bath, the DNA was pelleted by centrifugation.

The DNA pellet was dissolved in 10 µl 10X BglII buffer, 5 µl (∼50 units) restriction enzyme BglII, and 85 µl H₂0, and the reaction was placed at 37°C for 2 hours. After the BglII digestion, the reaction mixture was loaded onto a 0.85% agarose gel, and the fragments were separated by electrophoresis. After inspecting the gel stained with ethidium bromide under ultraviolet light, the band containing the desired ∼4.2 kb HindIII-BglII fragment was excised from the gel, and extracted as described in Example 4A. The pellet was resuspended in 10 µl of dH₂0 and constituted ∼5 µg of the desired ∼4.2 kb HindIII-BglII restriction fragment of plasmid pSV2-β-globin.

B.   Isolation of the ~2.0 kb HindIII-BamHI Restriction
Fragment of Plasmid pTPA103.

The isolation of the desired ∼2.0 kb HindIII-BamHI restriction fragment of plasmid pTPA103 from Example 2 was accomplished in substantial accordance with the above teaching. The ∼5 ug of DNA obtained were suspended in 10 µl of distilled water and stored at -20°C.

### C. Ligation of Fragments to Construct Intermediate Plasmid pTPA301.

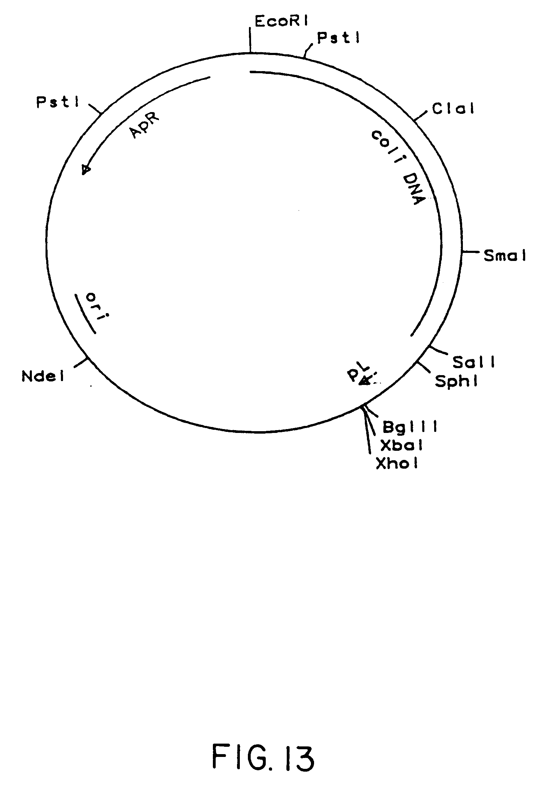

Two µl of the ∼4.2 kb BglII-HindIII restriction fragment of plasmid pSV2-β-globin, 4 µl of the ∼2.0 kb HindIII-BamHI fragment of plasmid pTPA103 were mixed together and then incubated with 2 µl 10X ligase buffer, 2 µl 10 mM ATP, 1 µl T4 DNA ligase (∼10 units), and 9 µl of H₂O at 4°C overnight. The ligated DNA constitutes the desired plasmid pTPA301; a restriction site and function map of the plasmid is presented in Figure 13 of the accompanying drawings.

D.    Construction of E. coli K12 RR1/pTPA301

The desired E. coli RR1/pTPA301 transformants were constructed in substantial accordance with the teaching of Example 2B. Plasmid DNA was obtained from the E. coli K12 RR1/pTPA301 transformants in substantial accordance with the procedure for plasmid pTPA102 DNA isolation.

E.   Construction of a BamHI Recognition Sequence on
Plasmid pSV2-dhfr

Ten µg of plasmid pSV2-dhfr (isolated from E. coli K12 HB101/pSV2-dhfr, ATCC 37146) were mixed with 10 µl 10X PvuII salts, 2 µl (∼20 units) PvuII restriction enzyme, and 88 µl of H₂0, and the resulting reaction was incubated at 37°C for two hours. The reaction was terminated by phenol and chloroform extractions, after which the PvuII-digested plasmid pSV2-dhfr DNA was precipitated and collected by centrifugation.

BamHI linkers (5'-CGGATCCCG-3') were kinased and prepared for ligation by the following procedure. To 1 µg of linker in 5 µl H₂0 was added: 10 µl of 5X Kinase salts (300 mM Tris-HCl, pH 7.8; 50 mM MgCl₂; 25 mM DTT), 5 µl of 5 mM ATP, 5 µl of BSA (1 mg/ml), 5 µl 10 mM spermidine, 19 µl of H₂0 and 1 µl of polynucleotide Kinase (10 units/µl). This reaction was then incubated at 37° for 60 minutes and stored at -20°C.

Five µl (∼5 µg) of the PvuII-digested plasmid pSV2-dhfr and 12 µl (∼.25 µg) of the kinased BamHI linkers were mixed and incubated with 15 µl of H₂0, 2 µl of 10X ligase buffer, 10 µl of 5 mM ATP, 2 µl of BSA (1 mg/ml), 2 µl of 10 mM spermidine, and 2 µl of T4 DNA ligase (∼ 2 units), at 16°C overnight.

Ten µl of 10X BamHI reaction buffer, 10 µl (∼ 150 units) of BamHI restriction enzyme, 2 µl of BSA (1 mg/ml) and 27 µl of H₂0 were added to the reaction, which was then incubated at 37°C for 3 hours. The reaction was loaded onto a 1% agarose gel, and the desired ∼1.9 kb fragment was isolated in substantial accordance with the teaching of Example 2A. All linker additions performed in these examples were routinely purified on an agarose gel to reduce the likelihood of multiple linker sequences in the resultant vector. The ∼3 µg of fragment obtained were suspended in 10 µl of TE buffer.

### F. Ligation

Next, approximately 15 µl (∼1 µg) of plasmid pTPA301 were digested with BamHI restricton enzyme as taught above. Since there is a unique BamHI site in plasmid pTPA301, this BamHI digestion generates a linear piece of DNA. The BamHI digested pTPA301 was ethanol precipitated and resuspended in 94 µl of dH₂0 and phosphatased using 1 unit calf intestinal alkaline phosphotase (Collaborative Research, Inc., 128 Spring Street, Lexington, MA 02173), and 5 µl 1 M Tris HCl pH 9.0 at 65° for 45 min. The DNA was then phenol: chloroform, chloroform:isoamyl alcohol extracted, ethanol precipitated and resuspended in 20 µl H₂0. Ten µl (∼.25 µg) of phosphotased pTPA301 was added to 5 µl BamHI-dhfr fragment (∼1.5 µg), 5 µl 10X ligase salts, 5 µl BSA (1 mg/ml)), 10 µl 5 mM ATP, 3 µl T4 ligase, and 12 µl H₂0. This reaction was incubated at 15°C overnight.

Plasmid pTPA303 was transformed into E. coli K12 RR1 (NRRL B-15210) in substantial accordance with the teaching of Example 2B and the resulting E. coli K12 RR1/pTPA303 transformants were identified by their ampicillin-resistant phenotype and by BamHI-HindIII restriction enzyme analysis of their plasmid DNA. Plasmid pTPA303 was isolated from the transformants in substantial accordance with the procedure of Example 1.

### Example 9

### Construction of Plasmid pBW32

A.    Isolation of the ~2700 bp EcoRI-BglII Fragment of
Plasmid pTPA303

About 10 µg of plasmid pTPA303 were digested to completion with 20 units BglII restriction enzyme in 1X BglII buffer (10 mM Tris-HCl pH 7.6, 50 mM NaCl, 10 mM MgCl₂) at 37°C. Tris-HCl concentration was adjusted to 110 mM for digestion with 20 units EcoRI restriction enzyme. The desired fragment was isolated and recovered in substantial accordance with the teaching of Example 4A.

B.    Isolation of the ~2340 bp EcoRI-HindIII Fragment of
Plasmid pTPA303

Plasmid pTPA303 was double-digested with HindIII and EcoRI restriction enzymes in substantial accordance with the teaching of Example 5A. The ∼2340 bp fragment was isolated and recovered as taught in Example 4A.

### C. Isolation of the ∼1990 bp Fragment from Plasmid pTPA303

Plasmid pTPA303 was double digested with HindIII and SstI restriction enzymes in HindIII buffer. The ∼1990 bp fragment was isolated and recovered as taught in Example 4A.

D.    Isolation of the ~680 bp XhoII-SstI Fragment of
Plasmid pBW28

About 10 µg of plasmid pBW28 were digested with XhoII enzyme to completion in XhoII buffer. The reaction was heated to 65°C for 10 minutes to heat inactivate the enzyme and then the DNA was recovered by ethanol precipitation. The DNA was subsequently digested with SstI enzyme to completion. The desired fragment was isolated and recovered as taught in Example 4A.

E.    Ligation and Transformation of E. coli MM294

About 0.1 µg each of the above fragments described in A through D were ligated in substantial accordance with the teaching of Example 4C. The ligation mix was used to transform E. coli MM294 as taught in Example 4D. Transformants were identified by their ampicillin resistant phenotype and restriction analysis of plasmid DNA.

### Example 10

### Construction of Plasmid pBW33

A.   ClaI Partial Digest of Plasmid pBW28

About 10 µg of plasmid pBW28 were diluted into 100 µl ClaI buffer (10mM Tris-HCl pH 7.9, 10 mM MgCl₂, 50 mM NaCl) in each of 3 tubes. Five units of ClaI restriction enzyme was added to each tube and incubations at 37°C done for 30 seconds, 1 minute, and 2 minutes, respectively. Reactions were stopped by the addition of EDTA to 25 mM. After chloroform extraction, the DNA was ethanol precipitated.

B.    Klenow Blunting of ClaI Ends

ClaI digests of pBW28 described in 14A were blunted by 4.5 units Klenow per sample in 50 mM Tris-HCl pH 7.6, 10 mM MgCl₂, 250 mM dATP, 250 mM dGTP, 250 mM dCTP, 250 mM TTP. The reaction was incubated at 20°C for 30 minutes, then stopped by heat inactivation of the enzyme. The DNA was recovered by ethanol precipitation.

The DNA was run on a 0.6% low-gelling-temperature agarose (Sea Plaque, FMC Corp., Marine Colloids, Rockland, ME 04841) gel for 2-3 hours at ∼130V and ∼75 mA. The gel was stained in a dilute solution of ethidium bromide, and the bands visualized under long wave UV light. The largest linear band, which was cut at only one of the two ClaI sites present on the plasmid DNA, was excised. The bands from all three samples were pooled and the DNA religated in substantial accordance with Methods In Enzymology 101:85 (Academic Press, Wu and Grossman, Eds.) except that the procedure was scaled down to 100 µl.

C.   Transformation of E. coli MM294

Competent E. coli MM294 were prepared and transformed with the ligation mix as taught in Example 4D except that 0.3 ml competent cells were used per 100 µl ligation mix and the procedure was scaled up accordingly. Transformants were identified by their tetracycline resistant phenotype and restriction digest analysis of plasmid DNA. The sequence of pBW33 differs from that of pBW28 as shown below. 
XbaI                                    1         2
5'-CTAGAGGGTATTAATAATGTATCGCGATTTAAATAAGGAGGAATAACA-3
    ''''''''''''''''''''''''''''''''''''''''''''''
    3'-TCCCATAATTATTACATAGCGCTAAATTTATTCCTCCTTATTGTAT-5'
                                                  NdeI
The insertion of two bases in the cistron changes the phasing so that stop 2 is used in pBW33 rather than the stop 1 used in pBW28.

### Example 11

### Construction of Plasmid pBW35

A.    Isolation of the 6.4 kb XbaI-SstI Fragment from pBW28

Plasmid pBW28 was digested with SstI restriction enzyme to completion. Buffer conditions were adjusted and the DNA was further digested to completion with XbaI. DNA was concentrated by ethanol precipitation and the ∼6.4 kb fragment was isolated and recovered as taught in Example 4A.

B.    Isolation of the ~700 bp SstI-NdeI Fragment from Plasmid
pBW28

Digestion and isolation of this fragment was performed as described in Example 11A except that NdeI restriction enzyme was substituted for XbaI.

C.   Construction of XbaI-NdeI Linker

The following linker was synthesized: 
XbaI
5'-CTAGAGGGTATTAAA-3'
3'-TCCCATAATTTAT-5'
After synthesis, ∼200 pmols of each linker were kinased in 20 µl reactions containing 50 mM Tris-HCl pH 7.6, 10 mM MgCl₂, 10 mM dithiothreitol, 2 nmols of ATP, 50 µCi of γ³²P-ATP (New England Nuclear (NEN) ) and 11 Richardson units T4 polynucleotide kinase for 30 minutes at 37°C. The enzyme addition and incubation steps were repeated. The reaction was terminated by heat inactivation of the enzyme at 70°C for 10 minutes. Phosphorylation efficiency was verified by descending paper chromatography on DE81 paper in 0.35M ammonium formate.

Following phosphorylation, aliquots from each reaction were mixed, heated at 90°C for 2 minutes and slow cooled in the water bath for 5 hours to room temperature to allow for annealing of strands.

D.    Ligation and Transformation into E. coli MM294

The fragments and linker described above were ligated in substantial accordance with the teaching of Example 10B using ∼50 pmols annealed linker in a 100 µl reaction. E. coli MM294 was transformed with the ligated material as taught in Example 10C. Transformants were identified by their tetracycline resistant phenotype and restriction digest analysis of plasmid DNA. Although the linker will ligate with another NdeI-digested fragment, the NdeI restriction site will not be regenerated upon ligation. Thus, plasmid pBW35 does not contain an NdeI site common to both plasmids pBW33 and pBW36.

### Example 12

### Construction of Plasmid pBW36

Plasmid pBW36 was constructed exactly as that taught for plasmid pBW35 except that the following linker was substituted for the linker identified in Example 11C:

5'-CTAGAGGGTATTACA-3'
' ' ' ' ' ' ' ' ' ' ' ' '
3'-TCCCATAATGTAT-5'
NdeI

### Example 13

Transformation of E. coli K12 RV308

About 0.1 µg each of plasmid pBW33, pBW35, and pBW36 DNA were transformed into E. coli RV308 in substantial accordance with the teaching of Example 2B, except that the competent cells were stored in 20% glycerol at -90°C prior to transformation.

The transformants were identified by their tetracycline resistant phenotype and by restriction enzyme analysis of plasmid DNA. The resultant cells were used to isolate their respective plasmid DNA in substantial accordance with the procedure of Example 1.

### Example 14

### Construction of Runaway Replicon-containing Plasmids

### A. Isolation of a Runaway Replicon-containing Fragment

Plasmid pCZ106 (NRRL B-15959) is double-digested with the restriction enzymes EcoRI and BamHI in substantial accordance with the teaching of Example 7A. The desired ∼10 kb EcoRI-BamHI vector fragment can be isolated in accordance with the teaching of Example 4A.

### B. Isolation of the Promoter-Containing Fragment

Plasmid pL110 is double-digested with EcoRI and XbaI restriction enzymes. The desired ∼1.9 kb fragment can be isolated and recovered as taught in Example 7B.

C. Isolation of the ~1.3 kb XbaI-BamHI Fragment

Plasmids pBW35 and pBW36 were individually double digested in separate reactions with XbaI and BamHI restriction enzymes. The desired fragments were isolated and recovered as taught in Example 7B.

In addition, one skilled in the art could isolate the ∼1.3 kb XbaI-BamHI fragment from plasmid pBW33 and substitute this fragment for the desired ∼1.3 kb XbaI-BamHI fragments from plasmids pBW35 and pBW36. The individual ∼1.3 kb XbaI-BamHI fragments from plasmid pBW35 and pBW36 were used in the subsequent ligation to construct plasmids pBW41 and pBW42 respectively.

### D. Ligations and Transformations

The ∼10 kb XbaI-BamHI vector, the ∼1.9 kb EcoRI-XbaI fragment from pL110, and the respective XbaI-BamHI fragments from plasmids pBW35 and pBW36 were individually ligated in substantial accordance with the teaching of Example 4C. The ligation mixture was split and used to transform E. coli K12 RV308/pRK248cIts and E. coli N5271 host cells in substantial accordance with the teaching of Example 4D except that both host strains were grown at 32°C rather than 37°C.

Plasmid pRK248cIts can be isolated from E. coli K12 JMB9/pRK248cIts, also known as E. coli K12 MCB3604, a strain deposited and made part of the permanent culture collection of the Northern Regional Research Laboratories. Plasmid pRK248cIts can be obtained from the NRRL under the accession number NRRL B-15631. Plasmid pRK248cIts comprises a temperature-sensitive mutant of the lambda pL repressor gene cI designated CI857; the construction of plasmid pRK248cIts is described in Bernard and Helinski, 1979, Methods of Enzymology 68:482, and Bernard et al., 1979, Gene 5:59. Plasmid pRK248cIts was isolated and purified in substantial accordance with the procedure of Example 1 and then transformed into E. coli K12 RV308 in substantial accordance with the procedure of Example 2B. Because plasmid pRK248cIts comprises a tetracycline resistance-conferring gene, and not the ampicillin resistance-conferring gene, tetracycline resistance was used as the basis for selection of the E. coli K12 RV308/pRK248cIts transformants.

However, a variety of plasmids could be used in place of plasmid pRK248cIts for the purposes of this example. Such plasmids would necessarily comprise a temperature-sensitive lambda pL repressor gene, a selectable marker independent of the ampicillin resistance-conferring gene, and a replicon, such as the replicon from plasmid pACYC184 or an R factor, that is compatible with the replicon of plasmid pBR322. Furthermore, temperature-sensitive control of the novel activating sequence of the present invention could also be obtained by using a host cell, such as E. coli N5271 (Lautenberger et al., 1983, Gene 23:75), that comprises a chromosomally-integrated, temperature-sensitive cI repressor gene.

The E. coli K12 RV308/pRK248cIts transformants and the E. coli N5271 host cells were made competent for transformation in substantial accordance with the procedure of Example 2B, except that the host cultures were grown at 32°C rather than 37°C. In separate transformations, plasmids pBW41 and pBW42 were transformed into E. coli K12 RV308/pRK248cIts and E. coli N5271 competent cells. Generally, it is believed that an expression plasmid need not carry the cI857 gene to repress the pL promoter, nor is it necessary to resort to heat inactivation to allow induction. As the copy number of the plasmid is increased, the repressor activity should be diluted.

The resulting E. coli N5271 transformants were then used to isolate their respective runaway replicon-containing plasmids pBW41 and pBW42.

### Example 15

### Construction of Runaway Replicon Plasmid pBW40

Plasmid pBW40 can be constructed substantially as taught for the construction of plasmids pBW41 and pBW42 except that the ∼1.3 kb XbaI-BamHI fragment from pBW33 is substituted for the similar XbaI-BamHI fragment from pBW35 or pBW36. After transformation into E. coli, the resulting transformants can then be used to isolate plasmid pBW40 DNA.

### Example 16

### Isolation of Plasmid pKC283

Lyophils of E. coli K12 BE1201/pKC283 are obtained from the Northern Regional Research Laboratory, Peoria, Illinois 61604, under the accession number NRRL B-15830. The lyophils are decanted into tubes containing 10 ml LB medium (10 g Bacto-tryptone, 5 g Bacto-yeast extract, and 10 g NaCl per liter; pH is adjusted to 7.5) and incubated two hours at 32°C, at which time the cultures are made 50 µg/ml in ampicillin and then incubated at 32°C overnight. The E. coli K12 BE1201/pKC283 cells were cultured at 32°C, because the cells comprise a temperature-sensitive cI repressor gene integrated into the cellular DNA. When cells that comprise a wild-type lambda pL repressor gene or do not comprise a lambda pL promoter are utilized in this plasmid isolation procedure, as described in subsequent Examples herein, the temperature of incubation is 37°C.

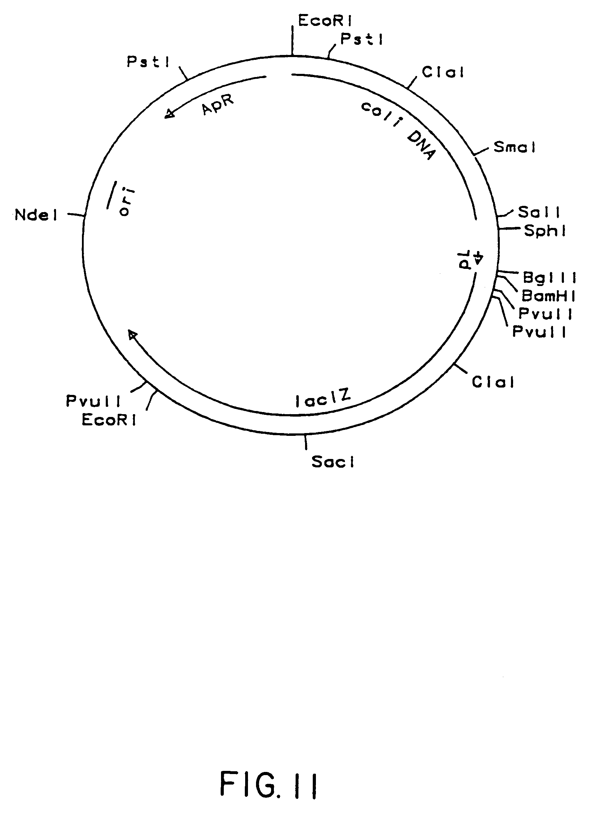

A small portion of the overnight culture is placed on LB-agar (LB medium with 15 g/l Bacto-agar) plates containing 50 µg/ml ampicillin in a manner so as to obtain a single colony isolate of E. coli K12 BE1201/pKC283. The single colony obtained was inoculated into 10 ml of LB medium containing 50 µg/ml ampicillin and incubated overnight at 32°C with vigorous shaking. The 10 ml overnight culture was inoculated into 500 ml LB medium and incubated at 32°C with vigorous shaking until the culture reached stationary phase. The plasmid DNA was isolated in accordance with the teaching of Example 1B. A restriction site and function map of plasmid pKC283 is presented in Figure 11 of the accompanying drawings.

### Example 17

### Construction of Plasmid pKC283PX

About 10 µl of the plasmid pKC283 DNA prepared in Example 20 were mixed with 20 µl 10 X medium-salt restriction buffer (500 mM NaCl; 100 mM Tris-HCl, pH 7.5; 100 mM MgCl₂; and 10 mM DTT), 20 µl 1 mg/ml BSA, 5 µl restriction enzyme PvuII (∼50 Units), and 145 µl of water, and the resulting reaction was incubated at 37°C for 2 hours. Restriction enzyme reactions described herein were routinely terminated by phenol and then chloroform extractions, which were followed by precipitation of the DNA, an ethanol wash, and resuspension of the DNA in TE buffer. After terminating the PvuII digestion as described above, the PvuII-digested plasmid pKC283 DNA was precipitated and then resuspended in 5 µl of TE buffer.

About 600 picomoles (pM) of XhoI linkers (5'-CCTCGAGG-3') were kinased in accordance with the teaching of Example 8E and then about 12.5 µl of the kinased XhoI linkers were added to 5 µl of PvuII-digested plasmid pKC283 DNA. Next, 2.5 µl of 10 X ligase buffer (300 mM Tris-HCl, pH 7.6; 100 mM MgCl₂; and 50 mM DTT), 2.5 µl of 1 mg/ml BSA, 7 µl of 5 mM ATP, 2.5 µl (about 2.5 units as defined by P-L Biochemicals) of T4 DNA ligase, 2.5 µl of 10 mM spermidine, and 3 µl of water were added to the DNA. The resulting ligation reaction was incubated at 4°C overnight. After the ligation reaction, the reaction mixture was adjusted to have the composition of high-salt buffer (0.1 M NaCl; 0.05 M Tris-HCl, pH 7.5; 10.0 mM MgCl₂; and 1 mM DTT). About 10 µl (100 units) of restriction enzyme XhoI were added to the mixture, and the resulting reaction was incubated at 37°C for 2 hours.

The reaction was terminated, and the XhoI-digested DNA was precipitated, resuspended, and ligated as described above, except that no XhoI linkers were added to the ligation mixture. The ligated DNA constituted the desired plasmid pKC283PX.

### Example 18

Construction of E. coli K12 MO(λ+)/pKC283PX

E. coli K12 MO(λ⁺)can be obtained from the Northern Regional Research Laboratories in lyophilized form under the accession number NRRL B-15993. E. coli K12 MO(λ⁺) comprises the wild-type lambda pL cI repressor gene, so that transcription from the hybrid pL-lpp promoter of the present invention does not occur in E. coli K12 MO(λ⁺) cells. The lyophils are reconstituted, single colonies of MO(λ⁺) are isolated, and a 10 ml overnight culture of the MO(λ⁺)cells is prepared in substantial accordance with the procedure of Example 20, except that the temperature of incubation is 37°C and no ampicillin is used in the growth media.

Fifty µl of the overnight culture were used to inoculate 5 ml of LB media which also contained 10 mM MgSO₄ and 10 mM MgCl₂. The culture was incubated at 37°C overnight with vigorous shaking. The following morning, the culture was diluted to 200 ml with LB media containing 10 mM MgSO₄ and 10 mM MgCl₂. The diluted culture was incubated at 37°C with vigorous shaking until the absorbance at 550 nm (A₅₅₀) was about 0.5, which indicated a cell density of about 1 x 10⁸ cells/ml. The culture was cooled for ten minutes in an ice-water bath, and the cells were then collected by centrifugation at 4000 g for 10 minutes at 4°C. The cell pellet was resuspended in 100 ml of cold 10 mM NaCl and then immediately re-pelleted by centrifugation. The cell pellet was resuspended in 100 ml of 30 mM CaCl₂ and incubated on ice for 20 minutes.

The cells were again collected by centrifugation and resuspended in 10 ml of 30 mM CaCl₂. A one-half ml aliquot of the cells was added to the ligated DNA prepared in Example 17; the DNA had been made 30 mM in CaCl₂. The cell-DNA mixture was incubated on ice for one hour, heat-shocked at 42°C for 90 seconds, and then chilled on ice for about two minutes. The cell-DNA mixture was diluted into 10 ml of LB media in 125 ml flasks and incubated at 37°C for one hour. One hundred µl aliquots were plated on LB-agar plates containing ampicillin and incubated at 37°C until colonies appeared.

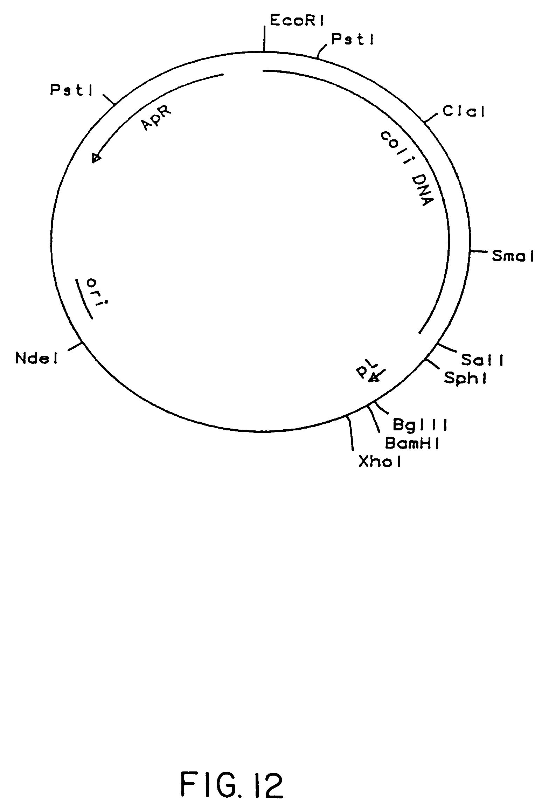

The colonies were individually cultured, and the plasmid DNA of the individual colonies was examined by restriction enzyme analysis and gel electrophoresis. Plasmid DNA isolation was performed on a smaller scale in accordance with the procedure of Example 16, but the CsCl gradient step was omitted until the desired E. coli K12 MO(λ⁺)/pKC283PX transformants were identified. A restriction site and function map of plasmid pKC283PX is presented in Figure 12 of the accompanying drawings.

### Example 19

Construction of E. coli K12 MO(λ+)/pKC283-L

Approximately ten µg of plasmid pKC283PX DNA prepared in accordance with the procedure of Example 16 were dissolved in 20 µl of 10X high-salt buffer, 20 µl 1 mg/ml BSA, 5 µl (∼50 units) restriction enzyme BglII, 5µl (∼50 units) restriction enzyme XhoI, and 150 µl of water, and the resulting reaction was incubated at 37°C for two hours. The reaction was stopped, and after precipitating the BglII-XhoI-digested DNA, the DNA was resuspended in 5 µl of TE buffer.

A DNA linker with single-stranded DNA ends characteristic of BglII and XhoI restriction enzyme cleavage was synthesized and phosphorylated. The linker was phosphorylated in substantial accordance with the procedure of Example 8E. The DNA linker had the following structure: 
5'-GATCTATTAACTCAATCTAGAC-3'
3'-ATAATTGAGTTAGATCTGAGCT-5'
The linker and BglII-XhoI-digested plasmid pKC283PX were ligated in substantial accordance with the procedure of Example 17. The ligated DNA constituted the desired plasmid pKC283-L. A restriction site and function map of plasmid pKC283-L is presented in Figure 13 of the accompanying drawings. The plasmid pKC283-L DNA was used to transform E. coli K12 MO(λ⁺) and the resulting E. coli K12 MO(λ⁺)/pKC283-L transformants were identified in substantial accordance with the procedure of Example 18.

### Example 20

Construction of E. coli K12 MO(λ+)/pKC283-LB

About 10 µg of plasmid pKC283-L DNA, prepared in substantial accordance with the procedures of Example 16, were dissolved in 20 µl 10X high-salt buffer, 20 µl 1 mg/ml BSA, 5 µl (∼50 units) restriction enzyme XhoI, and 155 µl of H₂0 were added, and the resulting reaction was incubated at 37°C for two hours. The XhoI-digested plasmid pKC283-L DNA was then precipitated from the reaction mixture by the addition of three volumes of 95% ethanol and one-tenth volume of 3 M sodium acetate, incubation in a dry ice-ethanol bath for five minutes, and centrifugation. The resulting DNA pellet was washed with 70% ethanol, dried, and resuspended in 2 µl 10X nick-translation buffer (0.5 M Tris-HCl, pH 7.2; 0.1 M MgSO₄; and 1 mM DTT), 1 µl of a solution 2 mM in each of the dNTPs, 15 µl of H₂0, 1 µl (∼6 units) of Klenow, and 1 µl of 1 mg/ml BSA. The resulting reaction was incubated at 25°C for 30 minutes; the reaction was stopped by incubating the solution at 70°C for five minutes.

BamHI linkers (5'-CGGGATCCCG-3') were phosphorylated and ligated to the XhoI-digested, Klenow-treated plasmid pKC283-L DNA in substantial accordance with the procedure of Example 17. After the ligation reaction, the DNA was digested with about 100 units of BamHI for about 2 hours at 37°C in high-salt buffer. After the BamHI digestion, the DNA was prepared for ligation in substantial accordance with the procedure of Example 17.

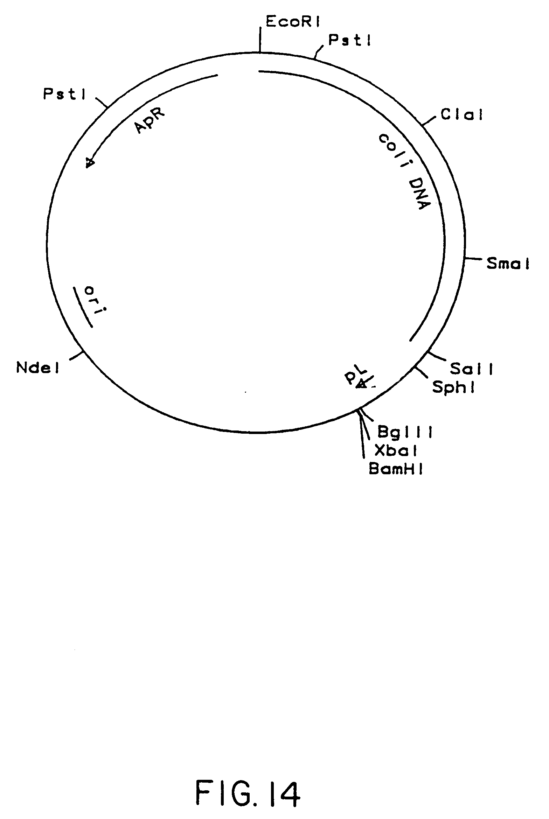

The ∼5.9 kb BamHI restriction fragment was circularized by ligation and transformed into E. coli K12 MO(λ⁺) in substantial accordance with the procedures of Examples 16 and 17. The E. coli K12 MO(λ⁺)/pKC283-LB transformants were identified, and then plasmid pKC283-LB DNA was prepared in substantial accordance with the procedure of Example 16. A restriction site and function map of plasmid pKC283-LB is presented in Figure 14 of the accompanying drawings.

### Example 21

Construction of E. coli K12 MO(λ+)/pL32

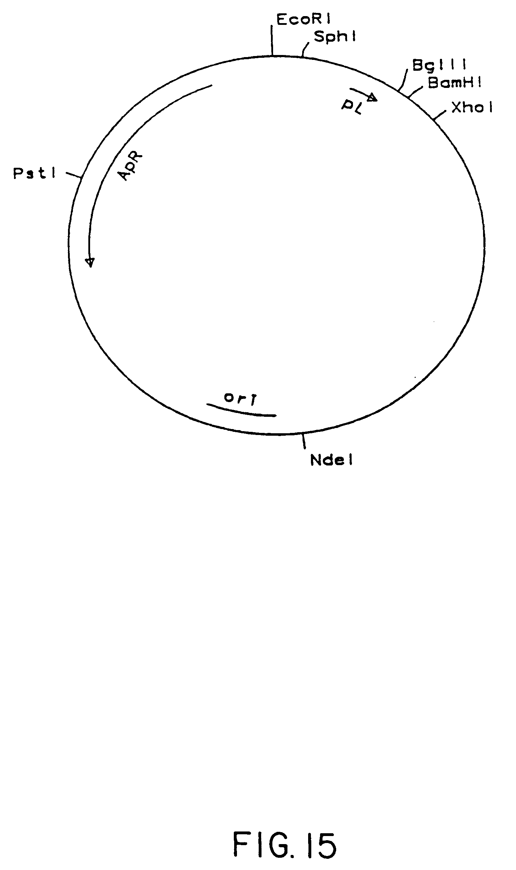

About 10 µg of plasmid pKC283PX were digested with restriction enzyme SalI in high-salt buffer, treated with Klenow, and ligated to EcoRI linkers (5'-GAGGAATTCCTC-3') in substantial accordance with the procedure of Example 20, with the exception of the starting plasmid, restriction enzymes, and linkers used. After digestion with restriction enzyme EcoRI, which results in the excision of ∼2.1 kb of DNA, the ∼4.0 kb EcoRI restriction fragment was circularized by ligation to yield plasmid pKC283PRS. The ligated DNA was used to transform E. coli K12 MO(λ⁺) in substantial accordance with the procedure of Example 18. After the E. coli K12 MO(λ⁺)/pKC283PRS transformants were identified, plasmid pKC283PRS DNA was prepared in substantial accordance with the procedure of Example 16. A restriction site and function map of plasmid pKC283PRS is presented in Figure 15 of the accompanying drawings.

About 10 µg of plasmid pKC283PRS were digested in 200 µl of high-salt buffer with about 50 units each of restriction enzymes PstI and SphI. After incubating the reaction at 37°C for about 2 hours, the reaction mixture was electrophoresed on a 0.6% low-gelling-temperature agarose gel for 2-3 hours at ∼130 V and ∼75 mA in Tris-Acetate buffer (0.04M Tris-acetate, 1mM EDTA).

The gel was stained in a dilute solution of ethidium bromide, and the band of DNA constituting the ∼0.85 kb PstI-SphI restriction fragment, which was visualized with long-wave UV light, was cut from the gel in a small segment. The volume of the segment was determined by weight and density of the segment, and an equal volume of 10 mM Tris-HCl, pH 7.6, was added to the tube containing the segment. The segment was then melted by incubation at 72°C. About 1 µg of the ∼0.85 kb PstI-SphI restriction fragment of plasmid pKC283PRS was obtained in a volume of about 100 µl. In an analogous manner, plasmid pKC283-LB was digested with restriction enzymes PstI and SphI, and the resulting ∼3.0 kb restriction fragment was isolated by agarose gel electrophoresis and prepared for ligation.

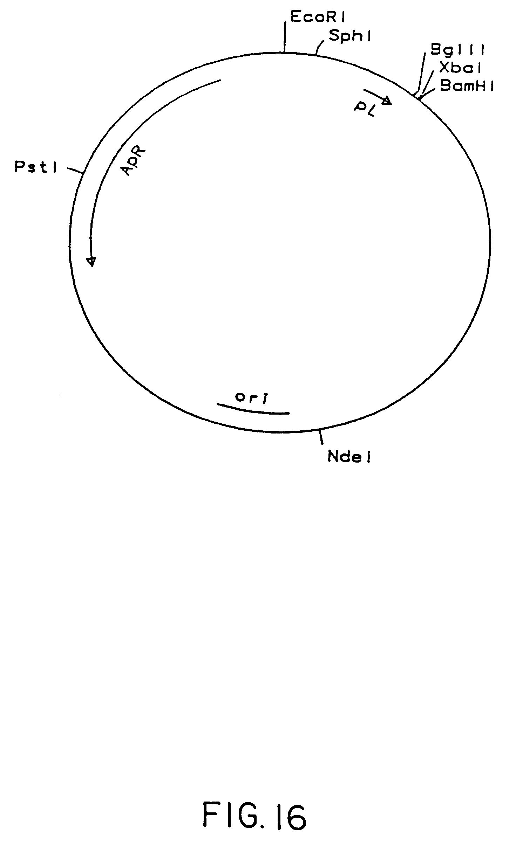

The ∼0.85 kb PstI-SphI restriction fragment of plasmid pKC283PRS was ligated to the ∼3.0 kb PstI-SphI restriction fragment of plasmid pKC283-LB in substantial accordance with the procedure of Example 17. The ligated DNA constituted the desired plasmid pL32. A restriction site and function map of plasmid pL32 is presented in Figure 16 of the accompanying drawings. Plasmid pL32 was transformed into E. coli K12 MO(λ⁺) cells in substantial accordance with the procedure of Example 18. Plasmid pL32 DNA was prepared from the E. coli K12 MO(λ⁺)/pL32 transformants in substantial accordance with the procedure of Example 16. Analysis of the plasmid pL32 DNA demonstrated that more than one EcoRI linker attached to the Klenow-treated, SalI ends of plasmid pKC283PX. The presence of more than one EcoRI linker does not affect the utility of plasmid pL32 or derivatives of plasmid pL32 and can be detected by the presence of an XhoI restriction site, which is generated whenever two of the EcoRI linkers are ligated together.

### Example 22

Construction of E. coli K12 MO(λ+)/pL47

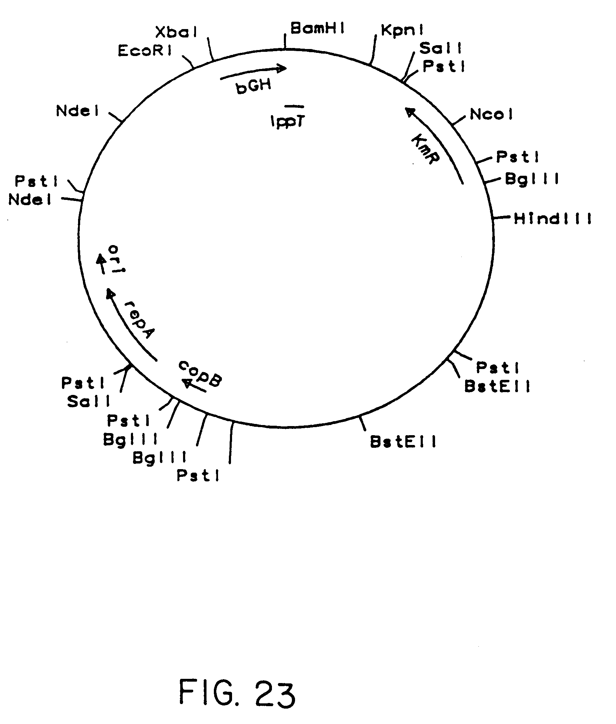

A restriction site and function map of plasmid pCC101 is presented in Figure 23 of the accompanying drawings. The construction of plasmid pCC101 is taught in Example 3 of U.S. Patent No. 4,745,069, issued May 7, 1988. To isolate the EK-BGH-encoding DNA, about 10 µg of plasmid pCC101 were digested in 200 µl of high-salt buffer containing about 50 units each of restriction enzymes XbaI and BamHI. The digestion products were separated by agarose gel electrophoresis, and the ∼0.6 kb XbaI-BamHI restriction fragment which encodes EK-BGH was isolated and prepared for ligation in substantial accordance with the procedure of Example 21.

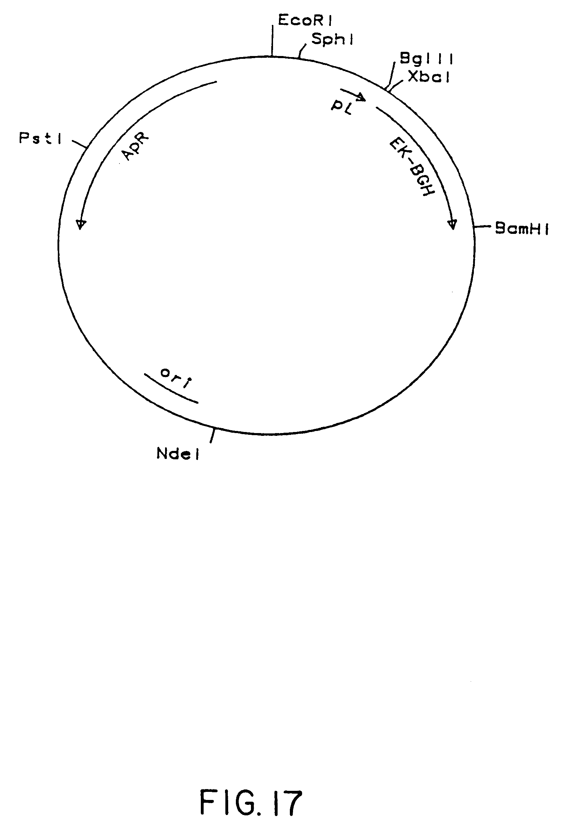

Plasmid pL32 was also digested with restriction enzymes XbaI and BamHI, and the ∼3.9 kb restriction fragment was isolated and prepared for ligation. The ∼3.9 kb XbaI-BamHI restriction fragment of plasmid pL32 was ligated to the ∼0.6 kb XbaI-BamHI restriction fragment of plasmid pCC101 in substantial accordance with the procedure of Example 17 to yield plasmid pL47. A restriction site and function map of plasmid pL47 is presented in Figure 17 of the accompanying drawings. Plasmid pL47 was transformed into E. coli K12 MO(λ⁺) in substantial accordance with the procedure of Example 18, and the E. coli K12 MO(λ⁺)/pL47 transformants were identified. Plasmid pL47 DNA was prepared from the transformants in substantial accordance with the procedures of Example 16.

### Example 23

Construction of E. coli K12 MO(λ+)/pL84

About 10 µg of plasmid pL32 were digested with about 50 units of restriction enzyme BamHI in 200 µl of high-salt buffer at 37°C for about two hours. The BamHI-digested plasmid pL32 DNA was precipitated, treated with Klenow, and ligated to SalI linkers (5'-CGTCGACG-3') in substantial accordance with the procedure of Example 20. After the SalI linkers were ligated, about 100 units of restriction enzyme SalI and about 50 units of restriction enzyme XbaI were added to the ligation mixture, which was adjusted to have the composition of high-salt buffer, and the resulting reaction was incubated at 37°C for two hours. The reaction products were separated by agarose gel electrophoresis, and the ∼3.9 kb SalI-XbaI restriction fragment was isolated and prepared for ligation in substantial accordance with the procedure of Example 21.

About 10 µg of plasmid pCC101 were digested with about 50 units each of restriction enzymes SalI and XbaI in 200 µl of high-salt buffer at 37°C for 2 hours. The reaction products were separated by agarose gel electrophoresis, and the ∼1.6 kb SalI-XbaI restriction fragment which encodes EK-BGH and the transcription terminator of the E. coli lpp gene was isolated and prepared for ligation in substantial accordance with the procedure of Example 21.

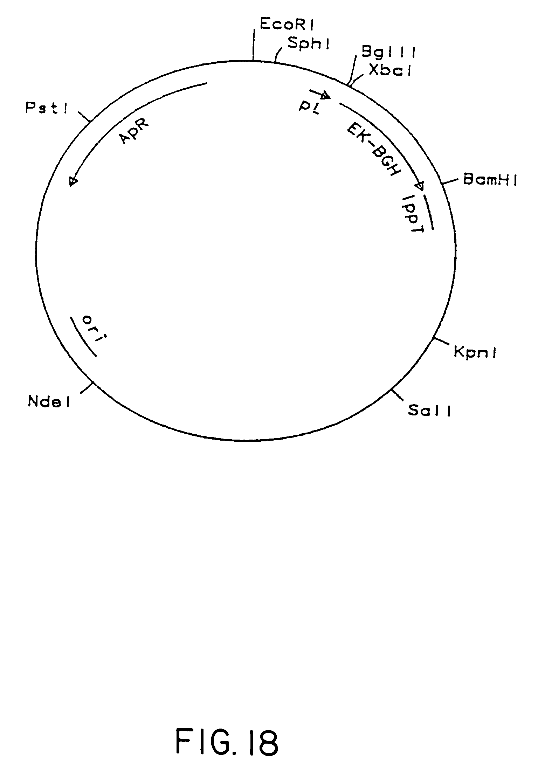

The ∼3.9 kb SalI-XbaI restriction fragment derived from plasmid pL32 was ligated to the ∼1.6 kb SalI-XbaI restriction fragment of plasmid pCC101 in substantial accordance with the procedure of Example 17. The ligated DNA constituted the desired plasmid pL84. A restriction site and function map of plasmid pL84 is presented in Figure 18 of the accompanying drawings. The ligated DNA was used to transform E. coli K12 MO(λ⁺) cells in substantial accordance with the procedure of Example 18. Plasmid pL84 DNA was prepared from the E. coli K12 MO(λ⁺)/pL84 transformants in substantial accordance with the procedure of Example 16.

### Example 24

Construction of E. coli K12 MO(λ+)/pL95

About 10 µg of plasmid pL84 were digested with about 50 units restriction enzyme NdeI in about 200 µl of high-salt buffer for two hours at 37°C. The NdeI-digested DNA was then precipitated and treated with Klenow in substantial accordance with the procedure of Example 20. The NdeI-digested, Klenow-treated plasmid pL84 DNA was then ligated to KpnI linkers (5'-GGGTACCC-3') in substantial accordance with the procedure of Example 17. After the linker ligation, the DNA was precipitated and the resulting DNA pellet was resuspended in 20 µl of 10X low-salt buffer (0.1 M Tris-HCl, pH 7.6; 0.1 M MgCl₂; and 10 mM DTT), 20 µl of 1 mg/ml BSA, 5 µl (about 50 units) restriction enzyme KpnI, and 155 µl of H₂0. The resulting reaction was incubated at 37°C for two hours.

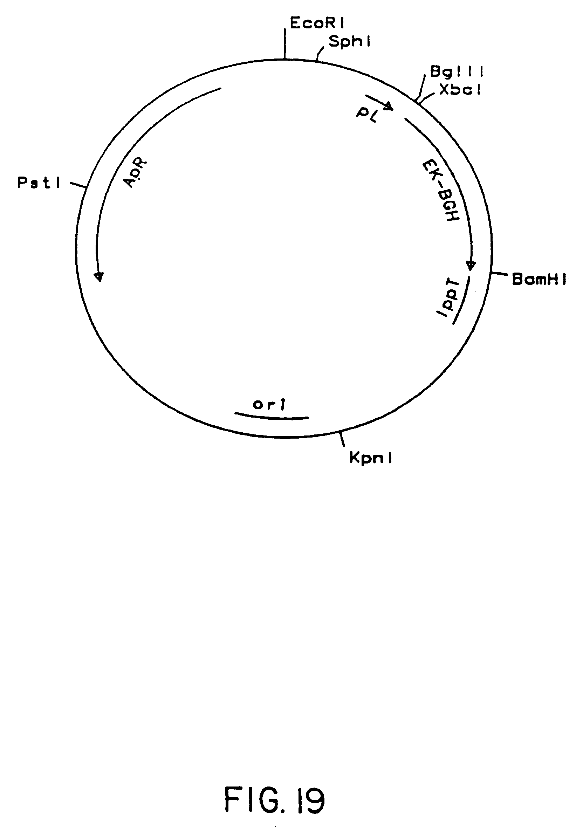

After the KpnI digestion, the DNA was loaded onto a low-melting-temperature agarose gel, and the ∼3.8 kb restriction fragment was isolated and recircularized by ligation in substantial accordance with the procedure of Example 21. The ligated DNA constituted plasmid pL95, which was used to transform E. coli K12 MO(λ⁺) in substantial accordance with the procedure of Example 22. A restriction site and function map of plasmid pL95 is presented in Figure 19 of the accompanying drawings.

### Example 25

Construction of E. coli K12 RV308/pPR12AR1

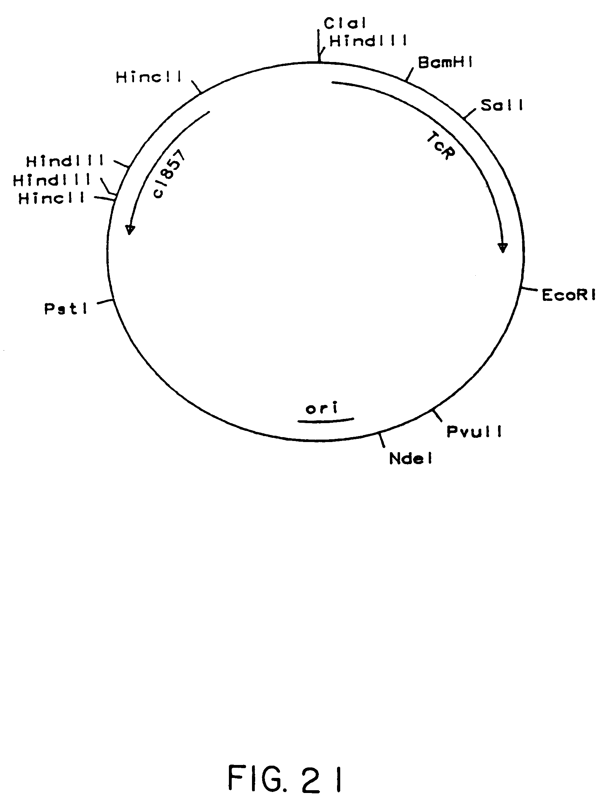

Plasmid pPR12 comprises the temperature-sensitive pL repressor gene cI857 and the plasmid pBR322 tetracycline resistance-conferring gene. Plasmid pPR12 is disclosed and claimed in U.S. Patent #4,436,815, which issued 13 March 1984. A restriction site and function map of plasmid pPR12 is presented in Figure 21 of the accompanying drawings.

About 10 µg of plasmid pPR12 were digested with about 50 units of restriction enzyme EcoRI in 200 µl of high-salt buffer at 37°C for two hours. The EcoRI-digested plasmid pPR12 DNA was precipitated and treated with Klenow in substantial accordance with the procedure of Example 20. After the Klenow reaction, the EcoRI-digested, Klenow-treated plasmid pPR12 DNA was recircularized by ligation in substantial accordance with the procedure of Example 17. The ligated DNA, which constituted the desired plasmid pPR12ΔR1, was used to transform E. coli K12 RV308 in substantial accordance with the procedure of Example 18 except that selection was based on tetracycline (5 µg/ml) resistance, not ampicillin resistance. E. coli K12 RV308 is available from the NRRL under the accession number NRRL B-15624. After the E. coli K12 RV308/pPR12ΔR1 transformants were identified, plasmid pPR12ΔR1 DNA was prepared from the transformants in substantial accordance with the procedure of Example 16.

About 10 µg of plasmid pPR12ΔR1 was digested with about 50 units of restriction enzyme AvaI in 200 µl of medium-salt buffer (BMB) at 37°C for 2 hours. The AvaI-digested plasmid pPR12ΔR1 DNA was precipitated and treated with Klenow in substantial accordance with the procedure of Example 20. After the Klenow reaction, the AvaI-digested, Klenow-treated plasmid pPR12ΔR1 DNA was ligated to EcoRI linkers (5'-GAGGAATTCCTC-3') in substantial accordance with the procedure of Example 17. After the linker ligation, the DNA was precipitated and then resuspended in about 200 µl of high-salt buffer containing about 50 units of restriction enzyme EcoRI. The resulting reaction was incubated at 37°C for about 2 hours. After the EcoRI digestion, the reaction mixture was loaded onto an agarose gel, and the ∼5.1 kb EcoRI restriction fragment was purified in substantial accordance with the procedure of Example 21. The ∼5.1 kb EcoRI restriction fragment was recircularized by ligation in substantial accordance with the procedure of Example 17. The ligated DNA constituted the desired plasmid pPR12AR1. The plasmid pPR12AR1 DNA was transformed into E. coli K12 RV308 in substantial accordance with the procedure of Example 18 except that selection was based on tetracycline resistance, not ampicillin resistance. After identifying the E. coli K12 RV308/pPR12AR1 transformants, plasmid pPR12AR1 DNA was prepared in substantial accordance with the procedure of Example 16. A restriction site and function map of plasmid pPR12AR1 is presented in Figure 21 of the accompanying drawings.

### Example 26

Construction of E. coli K12 RV308/pL110

About 10 µg of plasmid pPR12AR1 DNA were suspended in about 200 ml of high-salt buffer containing about 50 units each of restriction enzymes PstI and EcoRI, and the digestion reaction was incubated at 37°C for about 2 hours. The reaction mixture was then loaded onto an agarose gel, and the ∼2.9 kb PstI-EcoRI restriction fragment of plasmid pPR12AR1 was isolated and prepared for ligation in substantial accordance with the procedure of Example 21.

About 10 ug of plasmid pL47 were digested with restriction enzymes PstI and BamHI in 200 ul of high-salt buffer at 37°C for two hours. The PstI-BamHI digested DNA was loaded onto an agarose gel, and the ∼2.7 kb PstI-BamHI restriction fragment that comprised the origin of replication and a portion of the ampicillin resistance-conferring gene was isolated and prepared for ligation in substantial accordance with the procedure of Example 21. In a separate reaction, about 10 ug of plasmid pL47 DNA were digested with restriction enzymes EcoRI and BamHI in 200 ul of high-salt buffer at 37°C for two hours, and the ∼1.03 kb EcoRI-BamHI restriction fragment that comprised the novel transcriptional and translational activating sequence and the EK-BGH-encoding DNA was isolated and prepared for ligation in substantial accordance with the procedure of Example 21. The ∼2 ug of the ∼1.03 kb EcoRI-BamHI restriction fragment obtained were used in the construction of plasmid pL110.

The ∼2.7 kb PstI-BamHI and ∼1.03 kb EcoRI-BamHI restriction fragments of plasmid pL47 were ligated to the ∼2.9 kb PstI-EcoRI restriction fragment of plasmid pPR12AR1 to construct plasmid pL110, and the ligated DNA was used to transform E. coli K12 RV308 in substantial accordance with the procedure of Examples 17 and 18, except that tetracycline resistance, not ampicillin resistance, was used as the basis for selecting transformants.

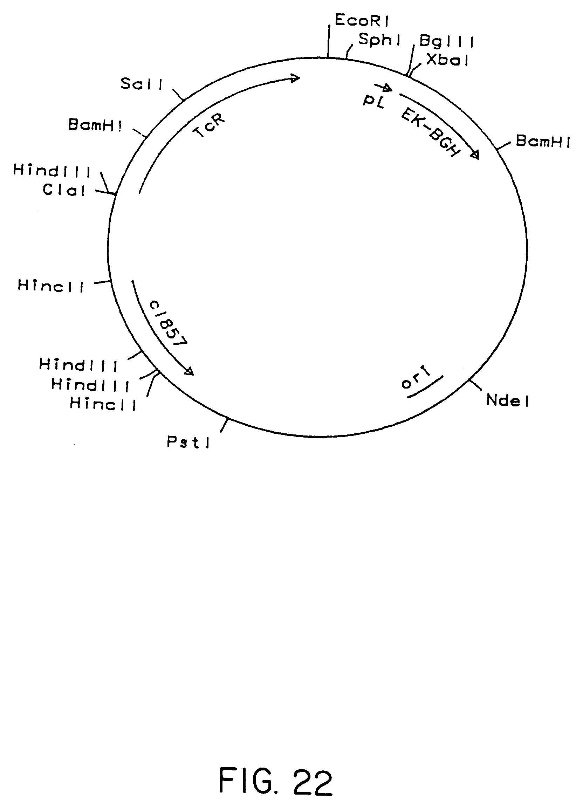

Two PstI restriction enzyme recognition sites are present in the EK-BGH coding region that are not depicted in the restriction site and function maps presented in the accompanying drawings. A restriction site and function map of plasmid pL110 is presented in Figure 22 of the accompanying drawings.

### Example 27

### Construction of Plasmid pBW46

### A. Construction of Plasmid pBW44

Plasmid pBW44 was constructed in a three-piece ligation reaction using restriction fragments from pBW33 and pL195. Two fragments from pBW33 were isolated which, upon religation, collectively reconstitute the plasmid except for the coding region of the carboxy-terminus of the t-PA gene. The third fragment, isolated from plasmid pL195, contains the coding region of the carboxy-terminus of the t-PA gene minus much of the 3'-non-coding sequence.

The ∼3.9 kb BamHI-ClaI and ∼2.7 kb ClaI-SstI restriction fragments from plasmid pBW33 were isolated in substantial accordance with the teaching of Example 4A substituting the appropriate restriction enzymes and buffers where necessary. In accordance, the ∼0.3 kb SstI-BamHI fragment from pL195 was isolated, mixed with the two pBW33 restriction fragments, and ligated in accordance with the teaching of Example 4C except that the final concentration of ATP was increased to 500 µM. The ligation product was used to transform E. coli MM294 cells in substantial accordance with the teaching of Example 1B except tetracycline at a concentration of 15 µg/ml was used and the transformants were incubated at 32°C.

### B. Construction of Plasmid mp8BW45

This vector was constructed from single-stranded pM8BW27 following the site specific mutagenesis procedure of Adelman et al., 1983, DNA 2(3):183-193 except that the S1 treatment was performed for 10 minutes at 20°C and the following synthetic oligodeoxyribonucleotide primer was substituted:
5'-GGATTTGCTGGGAAGTCCTGTGAAATATCCACC-3'
This primer was used to change nucleotide number 437 in CYS₈₃ from guanine to cytosine, thereby changing CYS₈₃ to SER₈₃.

The resulting mutagenesis mix was used to infect E. coli JM109 cells (commercially available from BRL) as taught in Example 5C. Desired mutants were identified by hybridization with the synthetic oligodeoxyribonucleotide 5'-GGGAAGTCCTGTGAAA-3'. Briefly, the filters were prepared as described by Hanahan and Meselson, 1980, Gene 10:63-67 except that Millipore 82 mm HATF filters were substituted and these filters were steamed instead of autoclaved.

Plaque lifts were performed by placing filters on top of prechilled (4°C for 60 minutes) overlay plates for 1-2 minutes. Next, the filters were transferred to Whatman 3MM filter paper saturated with the following solutions: 0.1 M NaOH, 1.5 M NaCl for 5 minutes and 0.5 M Tris pH 7.5, 3 M NaCl for an additional 5 minutes. The filters were air dried and then baked for 2 hours at 80°C. The filters were prehybridized at 45°C for 90 minutes in a solution of 6X SSC (0.9 M NaCl and 0.09 M Na citrate), 0.1% sodium pyrophosphate, 0.1% sodium dodecyl sulfate (SDS), 10X Denhardt's (0.2% ficoll, 0.2% polyvinylpyrrolidone, 0.2% BSA) and 1 µg/ml E. coli DNA.

Next, the filters were hybridized as follows. The radioactive probe (specific activity = 72 µCi/pmol) was diluted in hybridization buffer (6X SSC, 0.1% sodium pyrophosphate, 10X Denhardt's) to a concentration of 0.23 pmols/ml and added to the filter. The hybridization proceeded at 45°C for 2 hours. The filters were rinsed with 6X SSC according to the following schedule:

| No. of washes | Temperature (°C) | Time (minutes) |
|---|---|---|
| 1 | 25 | 2 |
| 2 | 25 | 20 |
| 1 | 37 | 10 |
| 2 | 50 | 3 |

The filters were exposed to X-ray film at -70°C for one hour with an intensifying screen. The DNA from the plaques which hybridized to the probe was extracted and identified by dideoxy sequencing.

### C. Final Construction

Plasmid pBW46 was constructed in a three-piece ligation using restriction fragments from pBW44 and mp8BW45. A fragment from mp8BW45 containing the section of t-PA encoding sequence wherein the cysteine substitution was made, was ligated to two fragments from plasmid pBW44 thereby forming a new gene sequence coding for a kringle-less form of t-PA that does not contain any free sulfhydryl residues.

The ∼4440 bp EcoRI-ClaI and ∼1950 ClaI-NdeI restriction fragments from pBW44 were isolated in substantial accordance with the teaching of Example 4A substituting the appropriate restriction enzymes and buffers where necessary. In accordance, the ∼562 bp NdeI-EcoRI fragment from mp8BW45 was isolated, mixed with the above fragments and ligated in substantial accordance with the teaching of Example 27. The ligation product was used to transform E. coli MM294 cells and the RF DNA was isolated in substantial accordance with the alkaline miniscreen procedure of Birnboim and Doly, 1979. E. coli K12 RV308 was transformed with this plasmid DNA for expression of the SER₈₃mt-PA3.

### Example 28

### Construction of Plasmid mp18BW50

### A. Construction of mp18BW47

The construction of this vector was performed substantially as taught in Example 5 with the conditions modified accordingly for the use of different restriction enzymes. Thus, the ∼7250 bp HindIII-SstI restriction fragment from mp18 (BRL) was ligated to the ∼1440 HindIII-SstI fragment from pTPA103. This ligated material was used to transfect E. coli JM109 cells (BRL) and the transfectants were cultured and single-stranded DNA was prepared for use as a starting material in the following mutagenesis example.

### B. Site Specific Mutagenesis

Single-stranded mp18BW47 was mutagenized in substantial accordance with the teaching of Example 6 except that the Klenow reaction was done at room temperature for 60 minutes, followed by 4 hours at 37°C, and then overnight at 4°C. In addition, the S1 treatment was for 20 minutes at 20°C. The synthetic oligodeoxyribonucleotide primer used was
5'-GGATTTGCTGGGAAGTCCTGTGAAATAGGAAACAGTGACTGCTAC-3'
to delete amino acid residues 87 through 175 of native t-PA.

The resulting mutagenesis mix was used to transfect E. coli JM109 cells and desired mutants were identified by hybridization with a synthetic oligodeoxyribonucleotide probe (5'-GTCCTGTGAAATAGGAA-3') according to the teaching of Example 27. These positives were further confirmed by M13 sequencing using the M13 sequencing primer (5'-GTGCCCCGAAGGATTTG-3') and the recombinant clones were designated mp18BW50.

### Example 29

### Construction of Plasmid pL219

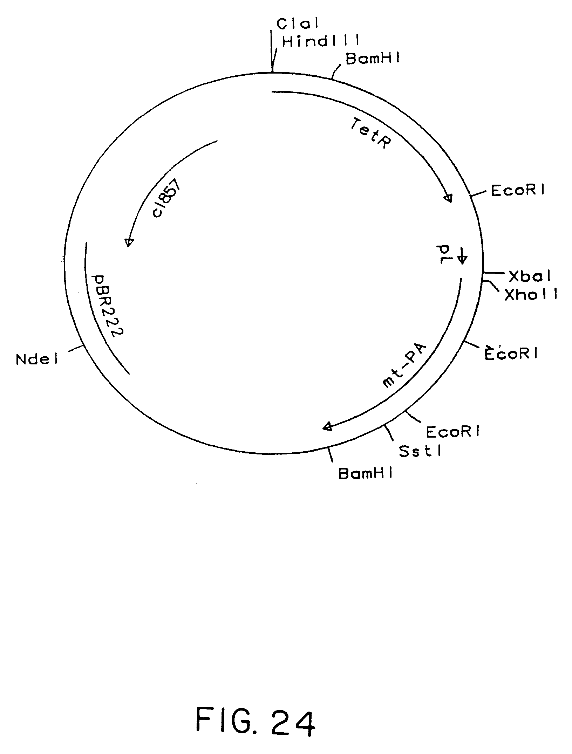

Plasmid pL219 is a procaryotic expression vector which contains a modified t-PA encoding gene sequence designated mt-PA4, wherein the first kringle (K1) domain, spanning amino acid residues 87 through 175, has been deleted and the CYS₈₃ residue has been changed to SER₈₃. To construct this derivative, restriction fragments from three previously constructed vectors were isolated and joined by T4 ligase in a multiple ligation reaction. Accordingly, the ∼1.0 kb BglII-SstI fragment comprising the 5' amino-terminus of the mt-PA4 gene, including the K1 domain deletion, was isolated from the replicative form of mp18BW50. The ∼50 bp XbaI-XhoII restriction fragment containing the two cistron construction was isolated from plasmid pBW33. These two fragments were mixed with the ∼6.1 kb SstI-XbaI fragment from pBW46, ligated and used to transform E. coli RV308. A restriction site and function map of plasmid pL219 is presented in Figure 24 of the accompanying drawings. Plasmid pL219 DNA was prepared from the E. coli RV308/pL219 transformants in substantial accordance with the procedure of Example 27.

### Example 30

### A. Site-Specific Mutagenesis of the TPA Coding Region and Construction of MP18BW52

About 5µg of plasmid pTPA103 (Example 2) in 10 µl of dH₂O are added to about 10 µl of 10X Hind III buffer and 80 µl of dH₂O. One µl (about 20 units) of restriction enzyme HindIII is added to the solution of plasmid pTPA103 DNA, and the resulting reaction is incubated at 37°C for 90 minutes. One µl (about 20 units) of restriction enzyme SstI and 10 µl of 1M Tris-HCl, pH = 7.6, is added to the solution of Hind III-digested plasmid pTPA103 DNA, and the resulting reaction is incubated at 37°C for 90 minutes. The Hind III-SstI-digested plasmid pTPA103 DNA is concentrated by ethanol precipitation, loaded onto a 1.5% agarose gel, and electrophoresed until the ∼1.4 kb HindIII-SstI restriction fragment is separated from the other digestion products. About 0.5 µg of the ∼1.4 bp Hind III-SstI restriction fragment is isolated from the gel, prepared for ligation, and resuspended in 20 µl of dH₂O.

About 4.5 µg of the replicative form (RF) of M13mp18 DNA (available from NEB in 35 µl of dH₂O is added to 10 µl of 10X HindIII buffer and 55 µl of dH₂O. One µl (about 20 units) of restriction enzyme HindIII is added to the solution of M13mp18 DNA, and the resulting reaction was incubated at 37°C for 1 hour. One µl (about 20 units) of restriction enzyme SstI and 10 µl of 1M Tris-HCl, pH = 7.6, is added to the solution of HindIII-digested M13mp18 DNA, and the resulting reaction is incubated at 37°C for 1 hour. The HindIII-SstI-digested M13mp18 DNA is collected by ethanol precipitation, resuspended in preparation for agarose gel electrophoresis, and the large restriction fragment isolated by gel electrophoresis as described above. About 1 µg of the large HindIII-SstI restriction fragment of M13mp18, 2 µl of 10X T4 DNA ligase buffer, 12 µl of dH₂O and ∼1 µl (about 1 Weiss unit) of T4 DNA ligase is added to 3 µl of the ∼1.4 kb HindIII-SstI restriction fragment of plasmid pTPA103, and the resulting ligation reaction is incubated at 16°C overnight.

E. coli JM103 cells (available from BRL) are made competent and transfected with the ligation mix in substantial accordance with the procedure described in the BRL M13 Cloning/'Dideoxy' Sequencing Instruction Manual, except that the amount of DNA used per transfection is varied. Recombinant plaques are identified by insertional inactivation of the β-galactosidase α-fragment-encoding gene, which results in the loss of the ability to cleave X-Gal to its indigo-colored cleavage product. For screening purposes, several white plaques are picked into 2.5 ml of L broth, to which is added 0.4 ml of E. coli K12 JM103 in logarithmic growth phase, that are cultured in minimal media stock to insure retention of the F episome that carries proAB. One liter of L broth contains 10 g of Difco Bacto tryptone, 5 g of Difco yeast extract, 5 g of NaCl, and 1 g of glucose. The 2.5 ml plaque-containing cell suspensions are incubated in an air-shaker at 37°C for 8 hours. Cells from 1.5 ml aliquots are pelleted and RF DNA isolated in substantial accordance with the alkaline miniscreen procedure of Birnboim and Doly, 1979, Nuc. Acids Res. 7:1513. The remainder of each culture is stored at 4°C for stock. The desired phage, designated MP18BW47, contains the ∼1.4 kb HindIII-SstI restriction fragment of plasmid pBW25 (Example 4) ligated to the ∼7.2 kb EcoRI-HindIII restriction fragment of M13mp18.

About fifty ml of log phase E. coli JM103 are infected with MP18BW47 and incubated in an air-shaker at 37°C for 18 hours. The infected cells are pelleted by low speed centrifugation, and single-stranded MP18BW47 DNA is prepared from the culture supernatant by scaling up the procedure given in the Instruction Manual. Single-stranded MP18BW47 is mutagenized in substantial accordance with the teaching of Adelman et al., 1983 DNA 2(3): 183-193, except that the Klenow reaction is done at room temperature for 30 minutes, then at 37°C for 60 minutes, then at 10°C for 18 hours. In addition, the S1 treatment is done at 20°C, the salt concentration of the buffer is one-half that recommended by its manufacturer, and the M13 sequencing primer (BRL) is used. The synthetic oligodeoxyribonucleotide primer used to delete the coding sequence for amino acid residues 4 through 175 of native TPA is
5'-GGAGCCAGATCTTACCAAGGAAACAGTGACTGCTAC-3'
The resulting mutagenesis mix is used to transfect E. coli K12 JM103 in substantial accordance with the infection procedure described above. Desired mutants are identified by restriction enzyme analysis of RF DNA and by Maxam and Gilbert DNA sequencing for amino acid residues 4 through 175 of native TPA deleted, is designated MP18BW52.

### Example 31

### Construction of Plasmid pL231

### A. Preparation of Plasmid Components

Plasmid pL231 encodes a t-PA derivative designated t-PA-6. Plasmid pL231 was constructed from plasmids pBW33 (Example 10), pBW46 (Example 27) and pMP18BW52 (Example 29) as follows:
Plasmid pBW33 DNA was prepared from E. coli MM294/pBW33 in substantial accordance with the procedure of Example 1. Plasmid pBW33 was digested sequentially with XbaI and XhoII. XbaI was purchased from New England Biolabs. Approximately 20 U of XbaI were used to digest approximately 10 µl of plasmid pBW33 in a total digestion volume of 20 µl comprising 50 mM NaCl, 10 mM Tris-HCl (pH = 7.9), 10 mM MgCl₂, and 100 µg/ml bovine serum albumin for 1 hour at 37°C. The XbaI digestion mixture of plasmid pBW33 was then digested with ∼20 U of XhoII (Boehringer Mannheim) for an additional hour at 37°C. The restriction fragments resulting from digestion of plasmid pBW33 with XbaI and XhoII were separated by electrophoresis on 0.6% agarose gels. The ∼50 bp fragment was harvested from the gel, ethanol precipitated, dried under vacuum then resuspended in ∼10 µl of TE. The ∼50 bp XbaI-XhoII fragment comprises the double-cistron described in Example 7C.

Plasmid pBW46 DNA was prepared from E. coli RV308/pBW46 in substantial accordance with the procedure of Example 1. Approximately 10 µg of plasmid pBW46 was digested sequentially with XbaI and SstI to prepare an ∼4.7 kb restriction fragment. The XbaI digestion procedure was described above. Approximately 10U of SstI (BMB) was used to digest the ∼10 µg of plasmid pBW46 for 1 hour at 37°C in Boehringer Mannheim Core buffer. The ∼4.7 kb XbaI-SstI restriction fragment was recovered from a 0.6% agarose gel following electrophoresis of the digestion mixture. The ∼4.7 kb XbaI-SstI fragment of pBW46 was ethanol precipitated, dried under vacuum and resuspended in 10 µl of TE.

Plasmid mp18BW52 was prepared from E. coli K12 JM103/mp18BW51 in substantial accordance with the procedure of Example 1. mp18BW52 was double digested with BglII and SstI. BglII and SstI were obtained from BRL and the digestions were performed in accordance with directions supplied by the vendor. The ∼718 bp fragment was harvested from a 0.6% agarose gel following electrophoresis, ethanol precipitated and resuspended in 10 µl of TE.

### B. Ligation of Plasmid pL231 Components

Ligation of the ∼50 bp XbaI-XhoII fragment of plasmid pBW33, the ∼4.7 kb XbaI-SstI fragment of plasmid pBW46, and the ∼718 bp BglII-SstI fragment of mp18BW52 resulted in plasmid pL231. The sticky ends resulting from XhoII digestion of pBW33 and the BglII digestion of mp18BW52 are complimentary and thus only plasmid pL231 results from ligation of the aforementioned components. The ligation mixture contained ∼1.5 µl of the pBW33 XbaI-XhoII fragment, ∼10 µl of the pBW46 Xba I-SstI fragment, ∼10 µl of the mp18BW51 BglII-SstI fragment, 0.1 mg/ml BSA, 83 mM ATP, ∼3 U of T4 DNA ligase (NEB) and water was added to achieve a final volume of 60 µl. The ligation reaction proceeded overnight at 15°C.

### C. Construction of E. coli K12 RV308/pL231

The ligation mixture in Example 35B was used to transform competent E. coli K12. The resultant E. coli K12 RV308/pL231 were then cultured in 2X YT medium. 2X YT medium was prepared by dissolving 16 g of Bactotryptone, 10 g of Bacto-yeast extract, and 5 g of NaCl in 900 ml of distilled water. The pH of the medium was adjusted to 7.0 with NaOH after which the volume was adjusted to 1 l with H₂O. The medium was then sterilized by autoclaving it for 20 minutes at 15/1b/in². Plasmid pL231 is similar to the expression vectors used for t-PA3 (pBW46) and t-PA4 (pL219) in that they are all thermoinducible, lambda pL promoter driven expression systems which utilize tetracycline resistance as a selectable marker. Expression of the t-PA-6 and t-PA-4, or t-PA-3 requires only that the expression systems disclosed herein be cultured in 2X YT medium or other such medias as are well known to skilled artisans until a sufficient cell mass accumulates after which the temperature is increased to 42°C. It is preferable to add tetracycline to the medium to prevent the outgrowth of E. coli which lack the expression vectors and thus have lost antibiotic resistance.

### Example 32

### Sulfitolysis Procedure

An urea sulfitolysis solution is prepared as follows. Tris (6.06 g/l), potassium tetrathionate (3.93 g/l), anhydrous sodium sulfite (12.6 g/l), and EDTA (0.57g/l) are added to cold deionized urea (7M) and the solution is adjusted to pH 8.5. The granule derived protein solution was diluted with 11 of the sulfitolysis solution per gram of protein. This solution has then stirred gently overnight at 4°C.

### Example 33

### Ion Exchange Purification

Fast Flow Q (Pharmacia) chromatography was used for purification of sulfitolyzed t-PA-6. A flow rate of 40 ml/cm²/hour was used for the charge, wash, and elution phases of the chromatography. Prior to the purification process, the columns were equilibrated with 50mM Tris in 7M urea, pH 8.5, hereafter referred to as equilibration buffer.

Prior to chromatographic purification, the sulfitolyzed t-PA-6 filtrate was tested for conductivity and if necessary, the conductivity was reduced to 2500 uMHOS by dilution with 7M urea. When the conductivity was established in the preferred range of 2500 uMHOS, the pH was measured and adjusted to the preferred range of 8.45 - 8.55.

Samples were loaded onto the column and the column was then washed with one column volume of equilibration buffer. t-PA-6 was eluted from the column using a linear salt gradient of 0 - 0.6M NaCl in the equilibration buffer. Fractions were collected and if they contained a sufficient protein concentration were pooled. Fibrinolytic activity was determined using the fibrin plate assay.

### Example 34

### A Two-Step Protein Folding Procedure

The starting material for the first step in the t-PA-6 folding process was sulfitolized (Example 32) and ion-exchange purified (Example 33) t-PA-6. The sulfitolized t-PA-6 was diluted from the chromatographic elution solution (7M urea, 50mM Tris, and a variable concentration of NaCl) to a final concentration of approximately 10 µg/ml using 7M urea. Reagents were added to achieve the following final concentrations: 7M urea, 50mM Tris, 0.15M lysine monohydrochloride, 7mM cysteine monohydrochloride, 0.2M NaCl, 1mM Na₂EDTA and the pH was adjusted to 9.2 by addition of NaOH. This initial step in the refolding was allowed to proceed at about 4°C with gentle stirring for approximately 16 hours.

Step 2 of the folding process was initiated by 1:2 dilution of step 1 with 50mM Tris, 80mM lysine monohydrochloride, 2mM cysteine monohydrochloride and the folding solution was adjusted to pH 9.2. Step 2 of the folding process was stirred gently at approximately 4°C for about 16 hours.

### Example 35

### Comparison of Dialysis and Two-Step Folding Procedures on the Fold Efficiency of t-PA-6

The two-step method was performed in substantial accordance with the teachings of Example 33. For purposes of comparison, a sample of the mixture in Step 1 was dialyzed against a buffer consisting of 1 part of Step 1 buffer (7M urea, 50mM Tris, 0.15M lysine monohydrochloride, 7mM cysteine monohydrochloride, 0.2M NaCl, and 1mM Na₂EDTA, pH 9.2) and 2 parts step 2 buffer (50mM Tris, 80mM lysine monohydrochloride, and 2mM cysteine monohydrochloride, pH 9.2). An 8,000 MW cut-off dialysis tubing was used and dialysis occurred at 4°C for approximately 16 hours (overnight). Results of this example are presented in Table 1.

### Example 36

### Optimal Urea Concentrations in the 2-Step Folding Process

### A. Effect of Urea Concentrations in the First Step of the 2-Step Folding Process

The protein folding procedure used to establish optimal urea concentrations for each step of the two-step method was conducted in substantial accordance with the teaching of Example 34. Urea concentrations in the first step of 7M, 4M, and 1M were evaluated for their effect on fold efficiency. Each of the urea concentrations evaluated was formulated to also contain 50mM Tris, 0.15M lysine monohydrochloride, 7mM cysteine monohydrochloride, 0.2M NaCl, 1mM Na₂EDTA, pH 9.2. Upon completion of the first step, the samples of varying urea concentrations were then folded under the standard conditions of the second step.

### B. Effect of Urea Concentration in the Second Step of the Two-Step Folding Process

Ion-exchange purified, sulfitolyzed t-PA-6A was diluted to a final concentration of 6 ug/ml in 7M urea, 50mM Tris, 0.15M lysine monohydrochloride, 7mM cysteine monohydrochloride, 0.2M NaCl, 1mM Na₂EDTA, pH 9.2. This first step was allowed to incubate with gentle mixing at about 4°C for approximately 16 hours. Following the first step, the t-PA-6A mixture was diluted with various amounts of step 2 buffer: 50mM Tris, 80mM lysine monohydrochloride, 2mM cysteine monohydrochloride, pH 9.2 to achieve a range of urea concentrations. Urea concentrations evaluated for fold efficiency in the second step of the two-step method were: 0.14M, 0.35M, 0.56M, 0.70M, 1.05M, 1.4M, 1.75M, 2.1M, 2.45M, 2.8M, 3.15M, 3.5M, 3.85M, 4.2M, 4.55M, 4.9M, 5.25M, 5.6M, 5.95M, 6.3M, 6.65M, and 7.0M. Each of the samples containing the aforementioned urea concentrations also contained 50mM Tris, 80mM lysine monohydrochloride, and 2mM cysteine monohydrochloride. Thus all aspects of this example were performed in accordance with the teaching of Example 34 except that the various urea concentrations were evaluated in Step 2 of the folding process. Results of this study are presented in Figure 25.

## Claims

1. A method for folding tissue plasminogen activator and tissue plasminogen activator derivatives comprising kringle-2, which comprises adding an effective amount of lysine to the folding solution to increase the yield of substrate specific, enzymatically active molecules.

2. The method of Claim 1 wherein the lysine concentration is in the range of from about 0.01M to about 1.5M.

3. The method of Claim 2 wherein the lysine concentration is in the range of from about 0.02M to about 1M.

4. The method of Claim 3 wherein the lysine concentration is approximately 0.13M.

5. The method of Claim 4 wherein said tissue plasminogen activator derivative is t-PA-6.

6. The method of Claim 1 wherein said improved method further comprises: (a) preparing a solution of the t-PA or t-PA derivative in a sufficient concentration of urea to disrupt secondary and tertiary structure of said t-PA or t-PA derivative, and (b) diluting the step (a) solution to yield a second environment conducive to the further folding of the t-PA or t-PA derivative into substrate specific, enzymatically active molecules.

7. The method of Claim 7 wherein said urea concentration in step A is from about 5M to about 8M.

8. The method of Claim 7 wherein said urea concentration is about 7M.

9. The method of Claim 7 wherein the effective concentration of lysine is present only in the second step of the folding method.

10. The method of Claim 6 wherein the lysine concentration is approximately 0.13M.

11. The method of Claim 10 wherein said t-PA derivative is t-PA-6.
